(19) Europäisches Patentamt
European Patent Office
Office européen des brevets

(11) EP 4 268 797 A1

(12) EUROPEAN PATENT APPLICATION
published in accordance with Art. 153(4) EPC

(43) Date of publication:
**01.11.2023 Bulletin 2023/44**

(21) Application number: **21915337.6**

(22) Date of filing: **28.12.2021**

(51) International Patent Classification (IPC):
***A61K 6/891*** *(2020.01)* ***A61C 13/14*** *(2006.01)*
***A61C 13/15*** *(2006.01)* ***A61K 6/831*** *(2020.01)*
***A61K 6/871*** *(2020.01)*

(52) Cooperative Patent Classification (CPC):
**A61C 13/14; A61C 19/003; A61K 6/831; A61K 6/871; A61K 6/891**

(86) International application number:
**PCT/JP2021/048983**

(87) International publication number:
**WO 2022/145479 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2020 JP 2020219714**

(71) Applicant: **Kuraray Noritake Dental Inc.**
**Kurashiki-shi, Okayama 710-0801 (JP)**

(72) Inventors:
- **INOUE, Masashi**
  **Tainai-shi, Niigata 959-2653 (JP)**
- **ICHIKAWA, Seiya**
  **Tainai-shi, Niigata 959-2653 (JP)**

(74) Representative: **D Young & Co LLP**
**120 Holborn**
**London EC1N 2DY (GB)**

# (54) CURABLE COMPOSITION FOR DENTAL RESTORATION

(57) The present invention provides a dental restorative curable composition that has high mechanical strength and toughness and has excellent water resistance and smoothness/gloss durability in the form of a cured product, a dental composite resin comprising the dental restorative curable composition, and a dental mill blank comprising a cured product of the dental restorative curable composition. The present invention relates to a dental restorative curable composition comprising: a (meth)acrylic acid ester compound (A) which has at least one polymer structure selected from the group consisting of a polycarbonate, a polyarylate, and an aromatic polysulfone and in which terminal hydroxy residues derived from repeating units constituting the polymer structure are directly (meth)acryloylated; a (meth)acrylic acid ester compound (B) having two or more (meth)acryloyloxy groups (excluding the (meth)acrylic acid ester compound (A)); an inorganic filler (C) having an average primary particle diameter of 0.01 to 5 μm; and a polymerization initiator (D).

EP 4 268 797 A1

## Description

TECHNICAL FIELD

The present invention relates to a dental restorative curable composition that is suitably used as a dental material, particularly a dental composite resin, that can serve as a substitute for a part or the entirety of a natural tooth in the field of dentistry, a dental composite resin comprising the dental restorative curable composition, and a dental mill blank comprising a cured product of the dental restorative curable composition.

BACKGROUND ART

Dental restorative curable compositions composed of a polymerizable monomer, an inorganic filler, a polymerization initiator, etc., are called dental composite resins, and are dental materials most widely used today as materials for restoring lost parts and caries of teeth. Dental composite resins are required, in the form of a cured product obtained after polymerization curing, to have sufficient mechanical strength to serve as a substitute for natural teeth, water resistance that can withstand long-term intraoral restoration, polishability for obtaining a gloss equivalent to that of natural teeth, etc., and are required, in the form of paste before polymerization curing, to have handling properties suitable for performing operations of filling cavities using a dental instrument or the like.

For example, for the purpose of achieving excellent handling properties of paste and excellent wear resistance of a cured product, a technique of blending a polycarbonate dimethacrylate represented by the following formula (III) has been proposed (Patent Literature 1). Also, a photocurable resin composition comprising a polycarbonate having a molecular weight of 200 to 10,000 and having at least two (meth)acrylate groups in a molecule has been proposed (Patent Literature 2). When the polycarbonate is contained, the handling properties in the manufacture of casting clasps are excellent, surface characteristics are excellent, and deformability can be reduced.

[Chem. 1]

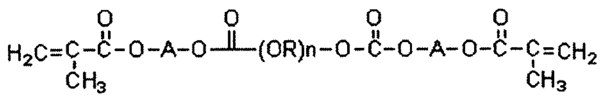

(III)

[where A is an alkylene group having 1 to 6 carbon atoms, R is an alkylene group having 2 to 5 carbon atoms and having at least two carbon atoms in a main chain thereof, and n is an integer from 1 to 4.]

Meanwhile, dental mill blanks are materials used for producing dental prosthesis such as inlays and crowns by a CAD/CAM system in which design is performed by a computer and machining is performed by a milling device, and the demand for dental mill blanks has increased rapidly in recent years. As a dental mill blank, a block body having a shape such as a rectangular parallelepiped, a cylinder, or a disc having an appropriate size is supplied and set on a cutting machine, and cut into a restoration having a crown shape, a dentition shape, or the like. Various materials such as glass ceramics, zirconia, titanium, acrylic resins, and composite materials comprising a polymer resin and an inorganic filler have been proposed as materials for dental mill blanks. Dental mill blanks are also required to have sufficient mechanical strength to serve as a substitute for natural teeth, water resistance that can withstand long-term intraoral restoration, and polishability for obtaining a gloss equivalent to that of natural teeth.

For example, to achieve mechanical strength and polishability, a method for producing a dental mill blank, in which an inorganic filler is press-molded to obtain a molded body and a polymerizable monomer is immersed in the molded body for thermal polymerization, is described (Patent Literature 3). This producing method allows dense filling with nanoparticles, thereby obtaining a dental mill blank having excellent mechanical strength and polishability.

CITATION LIST

Patent Literature

Patent Literature 1: JP S62-226907 A
Patent Literature 2: JP H5-78435 A
Patent Literature 3: WO 2014/021343

## SUMMARY OF INVENTION

### Technical Problem

However, as a result of studies conducted by the present inventors, it has been found that the dental curable composition disclosed in Patent Literature 1 has a problem of having low mechanical strength and water resistance. In addition, there is also room for improvement in terms of paste handling properties.

Moreover, the dental curable composition disclosed in Patent Literature 2 contains no inorganic filler, and thus has a problem that the paste handling properties when used as a dental composite resin are very poor and an obtained cured product has poor smoothness/gloss durability.

Furthermore, the dental mill blank disclosed in Patent Literature 3 has room for further improvement in terms of toughness (fracture energy), mechanical strength, and water resistance. Also, for example, in the application of dental composite resins, although the mechanical strength (especially flexural strength) increases when the content of an inorganic filler is increased, the toughness deceases when the blending amount of the inorganic filler is increased, but the toughness is excellent when the content of the inorganic filler is decreased. Therefore, the mechanical strength (especially flexural strength) and the toughness are known to have a trade-off relationship (Dental Materials and Devices, Vol. 7 No. 5 756-768, 1988).

The present invention has been made to solve the above problems of the conventional art, and aims to provide a dental restorative curable composition that has high mechanical strength and toughness and has excellent water resistance and smoothness/gloss durability in the form of a cured product. The present invention also aims to provide a dental restorative curable composition that also has excellent paste handling properties and a dental composite resin comprising the dental restorative curable composition. The present invention further aims to provide a dental mill blank that has excellent esthetics due to fewer air bubbles.

### Solution to Problem

As a result of intensive studies conducted to achieve the above aims, the present inventors have found that the above problems can be solved by using a (meth)acrylic acid ester compound in which the terminal hydroxy residues of a polycarbonate structure, a polyarylate structure, or an aromatic polysulfone structure, which are main chains, are directly (meth)acryloylated. The present inventors have conducted further studies based on the finding to complete the present invention.

That is, the present invention includes the following invention.

[1] A dental restorative curable composition comprising:

> a (meth)acrylic acid ester compound (A) which has at least one polymer structure selected from the group consisting of a polycarbonate, a polyarylate, and an aromatic polysulfone and in which terminal hydroxy residues derived from repeating units constituting the polymer structure are directly (meth)acryloylated;
> a (meth)acrylic acid ester compound (B) having two or more (meth)acryloyloxy groups (excluding the (meth)acrylic acid ester compound (A));
> an inorganic filler (C) having an average primary particle diameter of 0.01 to 5 μm; and
> a polymerization initiator (D).

[2] The dental restorative curable composition according to [1], wherein the polymer structure has a ring structure.

[3] The dental restorative curable composition according to [1] or [2], wherein the polymer structure comprises three or more functional groups that are each independently at least one functional group selected from the group consisting of carbonate groups, ester groups, and sulfonyl groups.

[4] The dental restorative curable composition according to any one of [1] to [3], wherein the (meth)acrylic acid ester compound (A) has a number average molecular weight of 300 to 5,000.

[5] The dental restorative curable composition according to any one of [1] to [4], wherein a content of the (meth)acrylic acid ester compound (A) is 1 to 40 mass% in a total amount of a polymerizable monomer.

[6] The dental restorative curable composition according to any one of [1] to [5], further comprising at least one compound selected from the group consisting of a mono(meth)acrylic acid ester compound (E-1) represented by the following general formula (I) and a mono(meth)acrylic acid ester compound (E-2) represented by the following general formula (II),

[Chem. 2]

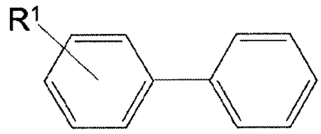

(I)

[Chem. 3]

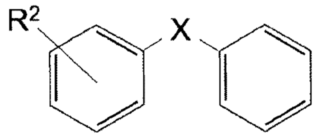

(II)

where $R^1$ and $R^2$ are each independently a group represented by the following general formula (i) or a group represented by the following general formula (ii), and X is a divalent hydrocarbon group having 1 to 6 carbon atoms, or an oxygen atom,

[Chem. 4]

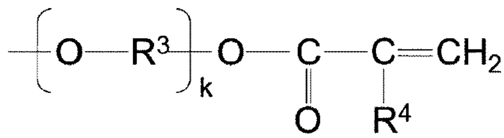

( i )

[Chem. 5]

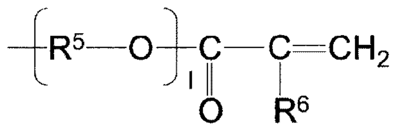

( ii )

where $R^3$ and $R^5$ are each independently a divalent hydrocarbon group having 1 to 10 carbon atoms, $R^4$ and $R^6$ are each independently a hydrogen atom or a methyl group, and k and l are each independently an integer from 0 to 6.

[7] The dental restorative curable composition according to [6], wherein X is an oxygen atom.
[8] The dental restorative curable composition according to [6] or [7], wherein k and l are each independently 0 or 1.
[9] The dental restorative curable composition according to any one of [1] to [8], wherein a content of the inorganic filler (C) is 50 to 95 mass% in a total amount of the dental restorative curable composition.
[10] The dental restorative curable composition according to any one of [1] to [9], wherein the polymerization initiator (D) comprises a photopolymerization initiator.
[11] The dental restorative curable composition according to any one of [1] to [10], wherein the polymerization initiator (D) comprises a thermal polymerization initiator.
[12] A dental composite resin comprising the dental restorative curable composition according to any one of [1] to [11].
[13] A dental mill blank comprising a cured product of the dental restorative curable composition according to any one of [1] to [11].

Advantageous Effects of Invention

According to the present invention, it is possible to provide a dental restorative curable composition that has high mechanical strength and toughness and has excellent water resistance and smoothness/gloss durability in the form of a cured product. The dental restorative curable composition of the present invention also has excellent paste handling properties. Furthermore, the dental restorative curable composition of the present invention can reduce air bubbles generated in a cured product thereof, and thus the cured product has excellent esthetics. Moreover, according to the present invention, even when the content of an inorganic filler is high (e.g., 50 to 95 mass% in the total amount of the

dental restorative curable composition), it is possible to provide a dental restorative curable composition that has high mechanical strength and toughness and has excellent water resistance and smoothness/gloss durability in the form of a cured product.

DESCRIPTION OF EMBODIMENTS

Hereinafter, the present invention will be described in detail. In the present specification, the upper limits and lower limits of numeric ranges (for example, contents of components, values and physical properties calculated from components, etc.) can be combined as appropriate. In the present specification, the numeric values of symbols in formulae can also be combined as appropriate.

The dental restorative curable composition of the present invention contains a specific (meth)acrylic acid ester compound (A) (hereinafter, sometimes referred to simply as "(meth)acrylic acid ester compound (A)"), a (meth)acrylic acid ester compound (B) having two or more (meth)acryloyloxy groups in one molecule, an inorganic filler (C) having an average primary particle diameter of 0.01 to 5 $\mu$m, and a polymerization initiator (D). To achieve the effects of the present invention, it is important that the (meth)acrylic acid ester compound (A) is a compound that has at least one polymer structure selected from the group consisting of a polycarbonate, a polyarylate, and an aromatic polysulfone and in which terminal hydroxy residues derived from repeating units constituting the polymer structure are directly (meth)acryloylated.

Although the reason why the effects of the present invention are achieved by the above configuration is not necessarily clear, the present inventors infer the reason as follows. Dental restorative curable compositions generally contain an inorganic filler, and a (meth)acrylic acid ester compound as a polymerizable monomer, and many cured products obtained therefrom have excellent mechanical strength such as flexural strength and elastic modulus. However, a polymer of the (meth)acrylic acid ester compound has low flexibility and is relatively brittle, and the brittleness is increased when the hardness is increased by blending the inorganic filler. Therefore, there is a toughness problem that the "fracture energy", which refers to the total energy applied until the cured product of the dental restorative curable composition is fractured, decreases, that is, material fracture is likely to occur due to the energy applied during occlusion. In order to increase the fracture energy (toughness), decreasing the blending amount of the inorganic filler or replacing the inorganic filler with an organic filler having high flexibility is considered, but the paste-like dental restorative curable composition mixed with the polymerizable monomer is likely to become sticky, and when filling a cavity with the dental restorative curable composition using a dental instrument, the dental restorative curable composition tends to stick to the instrument, resulting in poor handling properties. Furthermore, there is a problem that the cured product of the dental restorative curable composition has low water resistance and the physical properties of the cured product decrease with water absorption. As a factor for achieving both mechanical strength such as flexural strength and toughness, it is inferred that when the polymer structure of the polycarbonate, the polyarylate, or the aromatic polysulfone, which constitutes the (meth)acrylic acid ester compound (A), exhibits excellent toughness and the terminal hydroxy residues derived from the repeating units constituting the polymer structure are directly (meth)acryloylated, the mechanical strength is improved by the polymer structure being crosslinked so as to become dense during polymerization. The polymer structure also has excellent water resistance, and a densely crosslinked polymer obtained by polymerizing the (meth)acrylic acid ester compound (A) having the polymer structure is considered to have excellent water resistance. Furthermore, it is inferred that since the (meth)acrylic acid ester compound (A) has no extra spacer between the polymer structure and each methacryloyl group, the molecular weight can be reduced and the viscosity can be reduced to be low, so that the dental restorative curable composition has excellent handling properties and generation of air bubbles in a cured product thereof can be suppressed. Furthermore, by using the (meth)acrylic acid ester compound (A), the dental restorative curable composition also has excellent smoothness/gloss durability.

As the polymerizable monomer used in the present invention, any known polymerizable monomer that is used in dental restorative curable compositions (dental composite resins, etc.) is used without any limitations as long as a specific mass ratio is satisfied, but in general, radical polymerizable monomers are suitably used. Specific examples of the radical polymerizable monomers include: esters of $\alpha$-cyanoacrylic acid, (meth)acrylic acid, $\alpha$-halogenated acrylic acid, crotonic acid, cinnamic acid, sorbic acid, maleic acid, itaconic acid, etc.; (meth)acrylamide-based polymerizable monomers such as (meth)acrylamide and (meth)acrylamide derivatives; vinylesters; vinylethers; mono-N-vinyl derivatives; styrene derivatives; etc. Among them, one or more polymerizable monomers selected from the group consisting of (meth)acrylate-based polymerizable monomers and (meth)acrylamide-based polymerizable monomers are preferable, and (meth)acrylate-based polymerizable monomers are more preferable. The (meth)acrylate-based polymerizable monomers include the (meth)acrylic acid ester compound (A) and a (meth)acrylic acid ester compound (B) having two or more (meth)acryloyloxy groups which will be described later. In the present invention, the term "(meth)acrylic" is used to mean both methacrylic and acrylic, the term "(meth)acrylate" is used to mean both methacrylate and acrylate, and the term "(meth)acryloyloxy group" is used to mean both methacryloyloxy group and acryloyloxy group.

[(Meth)acrylic acid ester compound (A)]

The (meth)acrylic acid ester compound (A) is a compound that has at least one polymer structure selected from the group consisting of a polycarbonate, a polyarylate, and an aromatic polysulfone, in one molecule, and in which terminal hydroxy residues derived from repeating units constituting the polymer structure are directly (meth)acryloylated. As described above, to achieve the effects of the present invention, it is important that the (meth)acrylic acid ester compound (A) is a compound that has the polymer structure and in which the terminal hydroxy residues derived from the repeating units constituting the polymer structure are directly (meth)acryloylated. In contrast, as the conventional art, for example, in the compound represented by formula (III) described in Patent Literature 1, a spacer represented by "-O-A-O-" is present between each terminal hydroxy group in the polycarbonate and each methacryloyl group, the polycarbonate structure has a relatively low density, and the spacer itself is also an alkylene group, so that the mechanical strength (flexural strength) is considered to be lower than that of the present invention due to the flexible skeleton. Also, in the conventional art, in addition to the low density of the polymer skeleton, an alkylene group and an ether bond, a urethane bond, or the like are present between the methacryloyl groups and the hydroxy residues at both ends derived from repeating units constituting the polymer structure (e.g., PCUMA in Synthesis Example 6 described later corresponds thereto), so that it is considered that water is easily absorbed, resulting in lower water resistance than the present invention.

The specific structures of the polycarbonate, the polyarylate, and the aromatic polysulfone are not particularly limited, and examples of the polycarbonate include a polycarbonate derived from an aliphatic diol having 2 to 30 carbon atoms, a polycarbonate derived from an aromatic diol having 2 to 30 carbon atoms, and a polycarbonate derived from a combination of these diols. Examples of the polyarylate include amorphous polyarylates such as a polymer having an ester group and derived from a diol including a bisphenol skeleton and having 12 to 30 carbon atoms and a dicarboxylic acid having 4 to 14 carbon atoms. Examples of the aromatic polysulfone include a polymer derived from a diol having 2 to 30 carbon atoms and a dihalide having 2 to 30 carbon atoms and having a sulfonyl group. Examples of the aromatic polysulfone also include a polysulfone having repeating units represented by the following formula (1), a polyethersulfone having repeating units represented by the following formula (2), and a polyphenylsulfone having repeating units represented by the following formula (3).

$-O-Ar-C(CH_3)_2-Ar-O-Ar-SO_2-Ar-$ (1)

$-O-Ar-SO_2-Ar-$ (2)

$-O-Ar-Ar-O-Ar-SO_2-Ar-$ (3)

(where Ar represents a phenyl group (p-phenylene group) disubstituted at the para position, and the degree of polymerization and the molecular weight are not particularly limited).

Among them, the polycarbonate structure is preferable from the viewpoint that the cured product has excellent water resistance, toughness, smoothness/gloss durability, and handling properties. In addition, as the (meth)acrylic acid ester compound (A), one of these compounds may be used alone, or two or more of these compounds may be used in combination.

Also, the polymer structure of the (meth)acrylic acid ester compound (A) preferably includes a ring structure that is a rigid skeleton, from the viewpoint of excellent mechanical strength after curing. Examples of the ring structure include an alicyclic ring, an aromatic ring, and a heterocyclic ring.

Examples of the alicyclic ring include cyclopentane, cyclohexane, cycloheptane, dicyclodecane, tricyclodecane, adamantane, and isobornyl. Among them, tricyclodecane and isobornyl are more preferable.

Examples of the aromatic ring include benzene, naphthalene, anthracene, biphenyl, benzophenone, phenyl ether, and bisphenol A. Among them, biphenyl, benzophenone, phenyl ether, and bisphenol A are more preferable.

Examples of the heterocyclic ring include: heterocyclic rings having only a nitrogen atom as a heteroatom such as triazine, carbazole, pyrozirin, and piperidine; heterocyclic rings having only an oxygen atom as a heteroatom such as tetrahydrofuran, oxane, dioxane, dioxolane, and isosorbide; heterocyclic rings having an oxygen atom and a nitrogen atom as heteroatoms such as morpholine; heterocyclic rings having only a sulfur atom as a heteroatom such as tetrahydrothiophene and tetrahydrothiopyran; and heterocyclic rings containing a sulfur atom and a nitrogen atom as heteroatoms such as thiazine and thiazole. Among them, triazine, isosorbide, and morpholine are more preferable.

Also, from the viewpoint that the cured product has further excellent toughness, the polymer structure of the (meth)acrylic acid ester compound (A) preferably has three or more functional groups that are each independently at least one functional group selected from the group consisting of carbonate groups, ester groups, and sulfonyl groups, more preferably has four or more such functional groups, and further preferably has five or more such functional groups. For example, the polymer structure may be a structure having two or more carbonate groups and one or more sulfonyl groups, may be a structure having three or more carbonate groups, may be a structure having three or more ester

groups, or may be a structure having three or more sulfonyl groups.

The (meth)acrylic acid ester compound (A) contains the polymer structure, and the terminal hydroxy residues derived from the repeating units constituting the polymer structure are directly (meth)acryloylated in the (meth)acrylic acid ester compound (A). Here, the term "terminal hydroxy residues are directly (meth)acryloylated" refers to a state where no other atoms are present between the oxygen atoms included in the terminal hydroxy residues derived from the repeating units constituting the polymer structure and the carbon atoms included in the carbonyl groups in the (meth)acryloyl groups. The reaction for directly (meth)acryloylating the terminal hydroxy residues can be carried out according to a known method, and the method therefor is not particularly limited.

The (meth)acrylic acid ester compound (A) is preferably a compound in which a polymer structure is not crosslinked in a molecule.

The number average molecular weight (Mn) of the (meth)acrylic acid ester compound (A) is not particularly limited, and is preferably 300 to 5,000. From the viewpoint that the cured product has excellent toughness and the viscosity of the entire polymerizable monomer is reduced to be low to improve the handling properties of the dental restorative curable composition, the number average molecular weight (Mn) is more preferably 400 to 3,500 and further preferably 500 to 2,500. The number average molecular weight (Mn) in the present invention means a number average molecular weight determined by gel permeation chromatography (GPC) in terms of polystyrene, can be measured by a known method, and can be measured, for example, by the method described in EXAMPLES described later.

The content of the (meth)acrylic acid ester compound (A) is not particularly limited, and is preferably 1 to 40 mass% in the total amount of the polymerizable monomer. From the viewpoint of having more excellent mechanical strength and toughness, the content of the (meth)acrylic acid ester compound (A) is more preferably 3 to 35 mass% and further preferably 5 to 30 mass% in the total amount of the polymerizable monomer. The content of the (meth)acrylic acid ester compound (A) in the dental restorative curable composition of the present invention is not particularly limited, and is preferably 0.1 to 20 mass% in the total amount of the dental restorative curable composition. From the viewpoint of having more excellent mechanical strength and toughness, the content of the (meth)acrylic acid ester compound (A) is more preferably 0.2 to 15 mass% and further preferably 0.5 to 10 mass% in the total amount of the dental restorative curable composition. When the content of the (meth)acrylic acid ester compound (A) is 0.1 mass% or more in the total amount of the dental restorative curable composition, the toughness of the obtained cured product can be further improved. In addition, when the content of the (meth)acrylic acid ester compound (A) is 20 mass% or less in the total amount of the dental restorative curable composition, the mechanical strength of the obtained cured product can be further improved.

[(Meth)acrylic acid ester compound (B) having two or more (meth)acryloyloxy groups]

The (meth)acrylic acid ester compound (B) having two or more (meth)acryloyloxy groups in one molecule (hereinafter, sometimes referred to simply as "(meth)acrylic acid ester compound (B)"), which is used in the present invention, is not particularly limited as long as the (meth)acrylic acid ester compound (B) is a known (meth)acrylic acid ester compound that achieves the effects of the present invention and that is used in dental restorative curable compositions (dental composite resins, etc.). However, the (meth)acrylic acid ester compound (A) of the present invention is excluded therefrom. Hereinafter, specific examples of the (meth)acrylic acid ester compound (B) having two or more (meth)acryloyloxy groups will be described. As the (meth)acrylic acid ester compound (B), one compound may be used alone, or two or more compounds may be used in combination. The (meth)acrylic acid ester compound (B) imparts mechanical strength to the cured product.

(i) Bifunctional (meth)acrylic acid ester compound

Examples thereof include ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, 2,2-bis[4-[3-acryloyloxy-2-hydroxypropoxy]phenyl]propane, 2,2-bis[4-[3-methacryloyloxy-2-hydroxypropoxy]phenyl]propane (Bis-GMA), 2,2-bis[4-(meth)acryloyloxyethoxyphenyl]propane, 2,2-bis[4-(meth)acryloyloxypolyethoxyphenyl]propane, 1,2-bis[3-(meth)acryloyloxy-2-hydroxypropoxy]ethane, pentaerythritol di(meth)acrylate, [2,2,4-trimethylhexamethylenebis(2-carbamoyloxyethyl)]dimethacrylate (UDMA), and 2,2,3,3,4,4-hexafluoro-1,5-pentyl di(meth)acrylate. Triethylene glycol di(meth)acrylate, neopentyl glycol di(meth)acrylate, 1,6-hexanediol di(meth)acrylate, 1,10-decanediol di(meth)acrylate, Bis-GMA, 2,2-bis[4-methacryloyloxypolyethoxyphenyl]propane (average addition mole number of ethoxy group: 2.6), and UDMA are preferable.

(ii) Trifunctional or higher (meth)acrylic acid ester compound

Examples thereof include trimethylolpropane tri(meth)acrylate, trimethylolethane tri(meth)acrylate, tetramethylolmethane tri(meth)acrylate, pentaerythritol tetra(meth)acrylate, dipentaerythritol hexa(meth)acrylate, N,N'-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane.

[Mono(meth)acrylic acid ester compounds (E-1) and (E-2)]

From the viewpoint that the viscosity of the dental restorative curable composition can be made lower, good paste handling properties are imparted, and high mechanical strength and water resistance can be imparted to the cured product, the dental restorative curable composition of the present invention may contain at least one compound selected from the group consisting of a mono(meth)acrylic acid ester compound (E-1) represented by the following general formula (I) and a mono(meth)acrylic acid ester compound (E-2) represented by the following general formula (II).

[Chem. 6]

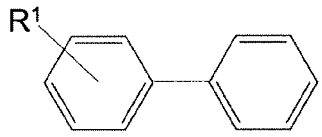

(I)

[Chem. 7]

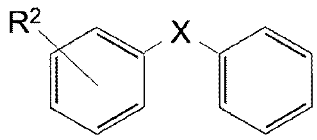

(II)

[where $R^1$ and $R^2$ are each independently a group represented by the following general formula (i) or a group represented by the following general formula (ii), and X is a divalent hydrocarbon group having 1 to 6 carbon atoms, or an oxygen atom.

[Chem. 8]

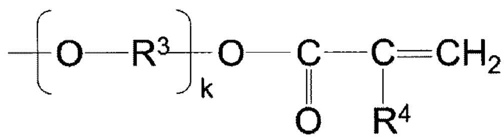

( i )

[Chem. 9]

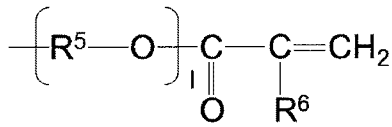

( ii )

(where $R^3$ and $R^5$ are each independently a divalent hydrocarbon group having 1 to 10 carbon atoms, $R^4$ and $R^6$ are each independently a hydrogen atom or a methyl group, and k and l are each independently an integer from 0 to 6.)].

In the mono(meth)acrylic acid ester compounds (E-1) and (E-2), the skeleton represented by the above general formula (I) and the skeleton represented by the above general formula (II) exhibit rigidity and hydrophobicity, so that the cured product of the obtained dental restorative curable composition has low water absorption and a decrease in mechanical strength can be suppressed. Hereinafter, the mono(meth)acrylic acid ester compound (E-1) and the mono(meth)acrylic acid ester compound (E-2) will be described.

The symbols in formula (I) will be described below. In formula (I), $R^1$ is a group represented by the above general formula (i) or a group represented by the above general formula (ii). From the viewpoint that the obtained dental

restorative curable composition has excellent curability, $R^4$ and $R^6$ in formula (i) or (ii) are each independently a hydrogen atom or a methyl group. $R^3$ and $R^5$ are each independently a divalent hydrocarbon group having 1 to 10 carbon atoms. From the viewpoint that the obtained dental restorative curable composition has good paste handling properties and has excellent mechanical strength after curing, the hydrocarbon group preferably has 1 to 6 carbon atoms, more preferably has 1 to 4 carbon atoms, and further preferably has 1 to 3 carbon atoms. Examples of the hydrocarbon group include: a linear or branched alkylene group having 1 to 10 carbon atoms; a cycloalkylene group having 3 to 10 carbon atoms; and a phenylene group. The symbols k and l are each independently an integer from 0 to 6. From the viewpoint that the obtained dental restorative curable composition has a low viscosity, can suppress generation of air bubbles in the cured product thereof, and has excellent curability, the symbol k is preferably 0 to 4, more preferably 0 to 3, further preferably 0 to 2, and particularly preferably 0 or 1. The symbol l is preferably 0 to 4, more preferably 0 to 2, and further preferably 0 or 1.

Examples of the mono(meth)acrylic acid ester compound (E-1) include o-phenylphenol(meth)acrylate, m-phenylphenol(meth)acrylate, p-phenylphenol(meth)acrylate, methoxylated-o-phenylphenol(meth)acrylate, methoxylated-m-phenylphenol(meth)acrylate, methoxylated-p-phenylphenol(meth)acrylate, ethoxylated-o-phenylphenol(meth)acrylate, ethoxylated-m-phenylphenol(meth)acrylate, ethoxylated-p-phenylphenol(meth)acrylate, propoxylated-o-phenylphenol(meth)acrylate, propoxylated-m-phenylphenol(meth)acrylate, propoxylated-p-phenylphenol(meth)acrylate, butoxylated-o-phenylphenol(meth)acrylate, butoxylated-m-phenylphenol(meth)acrylate, and butoxylated-p-phenylphenol(meth)acrylate. One of these compounds may be used alone, or two or more of these compounds may be used in combination. Among them, from the viewpoint that the obtained dental restorative curable composition has good paste handling properties and excellent mechanical strength after curing, ethoxylated-o-phenylphenol acrylate, ethoxylated-m-phenylphenol acrylate, ethoxylated-p-phenylphenol acrylate, propoxylated-o-phenylphenol acrylate, propoxylated-m-phenylphenol acrylate, and propoxylated-p-phenylphenol acrylate are more preferable, ethoxylated-o-phenylphenol acrylate, ethoxylated-m-phenylphenol acrylate, and ethoxylated-p-phenylphenol acrylate are further preferable, ethoxylated-o-phenylphenol acrylate and ethoxylated-m-phenylphenol acrylate are particularly preferable, and ethoxylated-o-phenylphenol acrylate is most preferable.

The symbols in formula (II) will be described below. In formula (II), X is a divalent hydrocarbon group having 1 to 6 carbon atoms, or an oxygen atom. From the viewpoint that the obtained dental restorative curable composition has good paste handling properties and excellent mechanical strength after curing, an oxygen atom is preferable. $R^2$ is a group represented by the above general formula (i) or a group represented by the general formula (ii). From the viewpoint that the obtained dental restorative curable composition has good paste handling properties and excellent mechanical strength after curing, $R^4$ and $R^6$ in formula (i) or (ii) are each independently a hydrogen atom or a methyl group. $R^3$ and $R^5$ are each independently a divalent hydrocarbon group having 1 to 10 carbon atoms. From the viewpoint that the obtained dental restorative curable composition has good paste handling properties and excellent mechanical strength after curing, the hydrocarbon group preferably has 1 to 6 carbon atoms, more preferably has 1 to 4 carbon atoms, and further preferably has 1 to 3 carbon atoms. Examples of the hydrocarbon group include a linear or branched alkylene group having 1 to 10 carbon atoms; a cycloalkylene group having 3 to 10 carbon atoms; and a phenylene group. Examples of the alkylene group include a methylene group, an ethylene group, an n-propylene group, an isopropylene group, an n-butylene group, and an n-pentylene group. Examples of the cycloalkylene group include a cyclobutylene group, a cyclopentylene group, a cyclohexylene group, and a cycloheptylene group. The symbols k and l are each independently an integer from 0 to 6. From the viewpoint that the obtained dental restorative curable composition has good paste handling properties and excellent mechanical strength after curing, the symbol k is preferably 0 to 4, more preferably 0 to 3, further preferably 0 to 2, and particularly preferably 0 or 1. The symbol l is preferably 0 to 4, more preferably 0 to 2, and further preferably 0 or 1.

Examples of the mono(meth)acrylic acid ester compound (E-2) include o-phenoxybenzyl(meth)acrylate, m-phenoxybenzyl(meth)acrylate, p-phenoxybenzyl(meth)acrylate, 2-(o-phenoxyphenyl)ethyl(meth)acrylate, 2-(m-phenoxyphenyl)ethyl(meth)acrylate, 2-(p-phenoxyphenyl)ethyl(meth)acrylate, 3-(o-phenoxyphenyl)propyl(meth)acrylate, 3-(m-phenoxyphenyl)propyl(meth)acrylate, 3-(p-phenoxyphenyl)propyl(meth)acrylate, 4-(o-phenoxyphenyl)butyl(meth)acrylate, 4-(m-phenoxyphenyl)butyl(meth)acrylate, 4-(p-phenoxyphenyl)butyl(meth)acrylate, 5-(o-phenoxyphenyl)pentyl(meth)acrylate, 5-(m-phenoxyphenyl)pentyl(meth)acrylate, 5-(p-phenoxyphenyl)pentyl(meth)acrylate, 6-(o-phenoxyphenyl)hexyl(meth)acrylate, 6-(m-phenoxyphenyl)hexyl(meth)acrylate, and 6-(p-phenoxyphenyl)hexyl(meth)acrylate. One of these compounds may be used alone, or two or more of these compounds may be used in combination. Among them, from the viewpoint that the obtained dental restorative curable composition has good paste handling properties and excellent mechanical strength after curing, o-phenoxybenzyl acrylate, m-phenoxybenzyl acrylate, p-phenoxybenzyl acrylate, 2-(o-phenoxyphenyl)ethyl acrylate, 2-(m-phenoxyphenyl)ethyl acrylate, and 2-(p-phenoxyphenyl)ethyl acrylate are more preferable, o-phenoxybenzyl acrylate, m-phenoxybenzyl acrylate, and p-phenoxybenzyl acrylate are further preferable, o-phenoxybenzyl acrylate and m-phenoxybenzyl acrylate are particularly preferable, and m-phenoxybenzyl acrylate is most preferable.

In the dental restorative curable composition of the present invention, the content of the mono(meth)acrylic acid ester compounds (E-1) and (E-2) is preferably 1 to 50 mass% with respect to the total amount of the polymerizable

monomer. From the viewpoint of having more excellent mechanical strength, water resistance, and paste handling properties, the content of the mono(meth)acrylic acid ester compounds (E-1) and (E-2) is more preferably 5 to 40 mass% and further preferably 10 to 30 mass% with respect to the total amount of the polymerizable monomer. As each of the mono(meth)acrylic acid ester compounds (E-1) and (E-2), one compound may be used alone, or two or more compounds may be used in combination.

As the polymerizable monomer contained in the dental restorative curable composition of the present invention, polymerizable monomers, such as mono(meth)acrylic acid ester compounds, (meth)acrylamide compounds, oxirane compounds, and oxetane compounds, other than the (meth)acrylic acid ester compound (A), the (meth)acrylic acid ester compound (B) having two or more (meth)acryloyloxy groups, and the mono(meth)acrylic acid ester compounds (E-1) and (E-2), can also be used. Examples of the polymerizable monomers include methyl (meth)acrylate, isobutyl (meth)acrylate, benzyl (meth)acrylate, lauryl (meth)acrylate, 2-(N,N-dimethylamino)ethyl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono(meth)acrylate, glycerin mono(meth)acrylate, erythritol mono(meth)acrylate, (meth)acryloyloxydodecylpyridinium bromide, (meth)acryloyloxydodecylpyridinium chloride, (meth)acryloyloxyhexadecylpyridinium chloride, (meth)acryloyloxydecylammonium chloride, 10-mercaptodecyl (meth)acrylate, N-methylol (meth)acrylamide, N-hydroxyethyl (meth)acrylamide, and N,N-bis(hydroxyethyl) (meth)acrylamide.

The polymerizable monomer contained in the dental restorative curable composition of the present invention may be substantially composed of only the (meth)acrylic acid ester compound (A), the (meth)acrylic acid ester compound (B) having two or more (meth)acryloyloxy groups, and the mono(meth)acrylic acid ester compounds (E-1) and (E-2). The polymerizable monomer being substantially composed of only the (meth)acrylic acid ester compound (A), the (meth)acrylic acid ester compound (B) having two or more (meth)acryloyloxy groups, and the mono(meth)acrylic acid ester compounds (E-1) and (E-2) means that the content of polymerizable monomers other than the (meth)acrylic acid ester compound (A), the (meth)acrylic acid ester compound (B) having two or more (meth)acryloyloxy groups, and the mono(meth)acrylic acid ester compounds (E-1) and (E-2), with respect to the total amount of the polymerizable monomer contained in the dental restorative curable composition, is less than 10.0 mass%, preferably less than 5.0 mass%, more preferably less than 1.0 mass%, further preferably less than 0.1 mass%, and particularly preferably less than 0.01 mass%.

The polymerizable monomer is preferably in liquid form, but may not necessarily be in liquid form at room temperature. Even when the polymerizable monomer is a solid polymerizable monomer, the polymerizable monomer can be mixed and dissolved in another liquid polymerizable monomer and be used.

The viscosity (25°C) of each polymerizable monomer is preferably 10 Pa·s or less, more preferably 5 Pa·s or less, and further preferably 2 Pa·s or less. Meanwhile, when two or more polymerizable monomers are mixed and used, or diluted in a solvent and used, the viscosity of each polymerizable monomer does not have to be in the above range, and the viscosity in a used state (mixed or diluted state) is preferably in the above range.

[Inorganic filler (C)]

As the inorganic filler (C) in the present invention, inorganic particles having an average primary particle diameter of 0.01 to 5 μm, which are used as fillers for dental composite resins, are used. By using such inorganic particles, the cured product has excellent smoothness/gloss durability and mechanical strength, and the paste handling properties are excellent. Examples of the inorganic particles include various types of glass (for example, glass containing boron and/or aluminum and various heavy metals with silicon dioxide (e.g., quartz, fused quartz, silica gel) or silicon as a main component), alumina, various types of ceramics, diatomaceous earth, kaolin, clay minerals (montmorillonite, etc.), activated clay, synthetic zeolite, mica, silica, calcium fluoride, ytterbium fluoride, calcium phosphate, barium sulfate, zirconium dioxide (zirconia), titanium dioxide (titania), and hydroxyapatite. As the inorganic filler (C), one of these inorganic fillers may be used alone, or two or more of these inorganic fillers may be used in combination.

Important physical properties desired for dental materials include transparency and radiopacity similar to those of natural teeth. Among these properties, the transparency can be achieved by matching the refractive index of the inorganic filler (C) and the refractive index of a polymer of the polymerizable monomer with each other as much as possible. Meanwhile, the radiopacity can be imparted by using an inorganic filler containing a heavy metal element such as zirconium, barium, titanium, lanthanum, or strontium (oxide or the like) as the inorganic filler (C). Such an inorganic filler containing a heavy metal element normally has a high refractive index within a range of 1.45 to 1.65. In the present invention, for example, a cured product of the (meth)acrylic acid ester compound (A), the (meth)acrylic acid ester compound (B), and the mono(meth)acrylic acid ester compound (E), which constitute the polymerizable monomer forming the polymer, normally has a refractive index within a range of 1.45 to 1.65. Thus, even combination with such an inorganic filler having radiopacity and having a high refractive index allows the difference in refractive index to be adjusted to be smaller, thereby improving the transparency of the obtained dental material.

Examples of the above inorganic filler having a high refractive index and capable of imparting radiopacity include barium borosilicate glass (for example, "E-3000" manufactured by Esstech, Inc., "8235", "GM27884", and "GM39923"

manufactured by SCHOTT, etc.), strontium boroaluminosilicate glass (for example, "E-4000" manufactured by Esstech, Inc., "G018-093" and "GM32087" manufactured by SCHOTT, etc.), lanthanum glass (for example, "GM31684" manufactured by SCHOTT, etc.), fluoroaluminosilicate glass (for example, "G018-091" and "G018-117" manufactured by SCHOTT, etc.), zirconia-containing glass (for example, "G018-310" and "G018-159" manufactured by SCHOTT, etc.), strontium-containing glass (for example, "G018-163", "G018-093", and "GM32087" manufactured by SCHOTT, etc.), zinc oxide-containing glass (for example, "G018-161" manufactured by SCHOTT, etc.), and calcium-containing glass (for example, "G018-309" manufactured by SCHOTT, etc.).

The shape of the inorganic filler (C) is not particularly limited, and fillers having various shapes such as a crushed shape, a sheet shape, a flake shape, a fiber shape (a short-fiber shape, a long-fiber shape, or the like), a needle shape, a whisker shape, and a spherical shape can be used. The inorganic filler (C) may be a combination of fillers having different shapes among the above shapes, as long as the requirements of the present invention are satisfied.

The average primary particle diameter of the inorganic filler (C) of the present invention is 0.01 to 5 μm. By using the inorganic filler (C) having an average primary particle diameter in this range, the dental restorative curable composition has excellent handling properties, and the cured product thereof has excellent smoothness/gloss durability and toughness. From such a viewpoint, the average primary particle diameter of the inorganic filler (C) is preferably 0.02 μm or more and more preferably 0.05 μm or more, and is further preferably 3 μm or less and even further preferably 2 μm or less. If the average primary particle diameter is less than 0.01 μm, the mechanical strength is likely to be impaired, and if the average primary particle diameter is larger than 5 μm, the brittleness may increase and the toughness may decrease.

The average primary particle diameter of the inorganic filler (C) can be determined by a laser diffraction scattering method or electron microscopy on particles. Specifically, the laser diffraction scattering method is convenient for measuring the particle diameter of particles of 0.1 μm or more, and the electron microscopy is convenient for measuring the particle diameter of particles of less than 0.1 μm. The laser diffraction scattering method may be adopted for determining whether or not the average primary particle diameter is 0.1 μm or more.

In the laser diffraction scattering method, the average primary particle diameter can be determined by, for example, performing measurement on a volume basis with a 0.2% sodium hexametaphosphate aqueous solution as a dispersion medium, using a laser diffraction particle size distribution analyzer (for example, "SALD-2300" manufactured by SHIMADZU CORPORATION).

In the electron microscopy, the average primary particle diameter can be determined by, for example, taking a micrograph of particles with a scanning electron microscope (SEM; for example, "SU3500H-800NA" manufactured by Hitachi High-Technologies Corporation), and measuring the particle diameters of particles (200 particles or more) observed in a unit field of view in the SEM image with image-analyzing particle size distribution measurement software (for example, "Mac-View" manufactured by Mountech Co., Ltd.). At this time, the particle diameter of a particle is determined as a circle-equivalent diameter that is the diameter of a circle having an area equal to that of the particle. The average primary particle diameter is calculated from the number of particles and the particle diameters thereof.

The content of the inorganic filler (C) in the dental restorative curable composition of the present invention is not particularly limited, and is preferably 50 to 95 mass%, more preferably 55 to 90 mass%, and further preferably 60 to 85 mass%, in the total amount of the dental restorative curable composition. When the content of the inorganic filler (C) is 50 mass% or more in the total amount of the dental restorative curable composition, the mechanical strength of the obtained cured product can be further improved. In addition, when the content of the inorganic filler (C) is 95 mass% or less in the total amount of the dental restorative curable composition, the toughness of the obtained cured product can be further improved.

The inorganic filler (C) in the present invention is preferably one subjected to a surface treatment with a surface treatment agent in advance. By using the surface-treated inorganic filler (C), the mechanical strength of the obtained dental restorative curable composition after curing can be further improved. In the case where two or more inorganic fillers (C) are used, only one of these inorganic fillers (C) may be subjected to a surface treatment, or these inorganic fillers (C) may all be subjected to a surface treatment. In the latter case, inorganic fillers (C) that have been individually subjected to a surface treatment may be mixed together, or a plurality of inorganic fillers that have been mixed together in advance may be collectively subjected to a surface treatment.

As the surface treatment agent, a known surface treatment agent can be used, and organometallic compounds such as organosilicon compounds, organotitanium compounds, organozirconium compounds, and organoaluminum compounds, and acidic group-containing organic compounds having at least one acidic group such as a phosphoric acid group, a pyrophosphoric acid group, a thiophosphoric acid group, a phosphonic acid group, a sulfonic acid group, or a carboxylic acid group can be used. In the case where two or more surface treatment agents are used, a surface treatment layer may be composed of a mixture of the two or more surface treatment agents, or may have a multilayer structure in which a plurality of surface treatment agent layers are stacked. The method for the surface treatment is not particularly limited, and a known method can be used.

Examples of the organosilicon compounds include compounds represented by $R^7_nSiY_{(4-n)}$ (where $R^7$ is a

substituted or unsubstituted hydrocarbon group having 1 to 12 carbon atoms, Y is an alkoxy group having 1 to 4 carbon atoms, an acetoxy group, a hydroxy group, a halogen atom, or a hydrogen atom, and n is an integer from 0 to 3, where each $R^7$ may be the same or different when a plurality of $R^7$s exist, and each Y may be the same or different when a plurality of Ys exist).

Specific examples of the organosilicon compounds include methyltrimethoxysilane, dimethyldimethoxysilane, phenyltrimethoxysilane, diphenyldimethoxysilane, methyltriethoxysilane, dimethyldiethoxysilane, phenyltriethoxysilane, diphenyldiethoxysilane, isobutyltrimethoxysilane, vinyltrimethoxysilane, vinyltriethoxysilane, vinyltris(β-methoxyethoxy)silane, 3,3,3-trifluoropropyltrimethoxysilane, methyl-3,3,3-trifluoropropyldimethoxysilane, β-(3,4-epoxycyclohexyl)ethyltrimethoxysilane, γ-glycidoxypropyltrimethoxysilane, γ-glycidoxypropylmethyldiethoxysilane, γ-glycidoxypropyltriethoxysilane, γ-methacryloyloxypropylmethyldimethoxysilane, γ-methacryloyloxypropylmethyldiethoxysilane, N-β(aminoethyl)-γ-aminopropylmethyldimethoxysilane, N-β(aminoethyl)-γ-aminopropyltrimethoxysilane, N-β(aminoethyl)-γ-aminopropyltriethoxysilane, γ-aminopropyltrimethoxysilane, γ-aminopropyltriethoxysilane, N-phenyl-γ-aminopropyltrimethoxysilane, γ-mercaptopropyltrimethoxysilane, trimethylsilanol, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, diethylsilane, vinyltriacetoxysilane, ω-(meth)acryloyloxyalkyltrimethoxysilane [having 3 to 12 carbon atoms between the (meth)acryloyloxy group and the silicon atom; e.g., γ-methacryloyloxypropyltrimethoxysilane], and ω-(meth)acryloyloxyalkyltriethoxysilane [having 3 to 12 carbon atoms between the (meth)acryloyloxy group and the silicon atom; e.g., γ-methacryloyloxypropyltriethoxysilane]. In the present invention, the term "(meth)acryloyloxy" is used to mean both methacryloyloxy and acryloyloxy.

Among them, coupling agents having a functional group that is copolymerizable with the polymerizable monomer, such as ω-(meth)acryloyloxyalkyltrimethoxysilane [having 3 to 12 carbon atoms between the (meth)acryloyloxy group and the silicon atom], ω-(meth)acryloyloxyalkyltriethoxysilane [having 3 to 12 carbon atoms between the (meth)acryloyloxy group and the silicon atom], vinyltrimethoxysilane, vinyltriethoxysilane, vinyltriacetoxysilane, and γ-glycidoxypropyltrimethoxysilane, are preferably used.

Examples of the organotitanium compounds include tetramethyl titanate, tetraisopropyl titanate, tetra-n-butyl titanate, butyl titanate dimmer, and tetra(2-ethylhexyl)titanate.

Examples of the organozirconium compounds include zirconium isopropoxide, zirconium-n-butoxide, zirconium acetylacetonate, and zirconyl acetate.

Examples of the organoaluminum compounds include aluminum acetylacetonate and an aluminum-organic acid salt chelate compound.

Examples of the acidic group-containing organic compounds having a phosphate group include 2-ethylhexyl acid phosphate, stearyl acid phosphate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides, alkali metal salts, and ammonium salts thereof.

As the acidic group-containing organic compounds having an acidic group such as a pyrophosphate group, a thiophosphate group, a phosphonate group, a sulfonate group, or a carboxylate group, for example, those described in WO 2012/042911 can be suitably used.

As the above surface treatment agent, one agent may be used alone, or two or more agents may be used in combination. Also, in order to increase the chemical bonding properties between the inorganic filler (C) and the polymerizable monomer to improve the mechanical strength of the cured product, an acidic group-containing organic compound having a functional group that is copolymerizable with the polymerizable monomer is more preferably used.

The use amount of the surface treatment agent is not particularly limited, and is, for example, preferably 0.1 to 50 parts by mass with respect to 100 parts by mass of the inorganic filler (C).

[Polymerization initiator (D)]

Next, the polymerization initiator (D) of the present invention will be described. Examples of the polymerization initiator (D) include a thermal polymerization initiator, a photopolymerization initiator, and a chemical polymerization initiator. One of these polymerization initiators may be used alone, or two or more of these polymerization initiators may

be used in combination.

Examples of the thermal polymerization initiator include organic peroxides and azo compounds.

Examples of the organic peroxides include ketone peroxides, hydroperoxides, diacyl peroxides, dialkyl peroxides, peroxyketals, peroxyesters, and peroxydicarbonates.

Examples of the ketone peroxides include methyl ethyl ketone peroxide, methyl isobutyl ketone peroxide, methyl cyclohexanone peroxide, and cyclohexanone peroxide.

Examples of the hydroperoxides include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoylperoxide, 2,4-dichlorobenzoyl peroxide, and lauroyl peroxide.

Examples of the dialkyl peroxides include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl-4,4-bis(t-butylperoxy)valerate.

Examples of the peroxyesters include $\alpha$-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butylperoxyhexahydroterephthalate, t-butylperoxy-3,3,5-trimethylhexanoate, t-butylperoxyacetate, t-butylperoxybenzoate, and t-butylperoxymaleic acid.

Examples of the peroxydicarbonates include di-3-methoxyperoxydicarbonate, di(2-ethylhexyl)peroxydicarbonate, bis(4-t-butylcyclohexyl)peroxydicarbonate, diisopropyl peroxydicarbonate, di-n-propyl peroxydicarbonate, di(2-ethoxyethyl)peroxydicarbonate, and diallyl peroxydicarbonate.

Among these organic peroxides, from the viewpoint of the overall balance among safety, storage stability, and radical generation capability, the diacyl peroxides are preferable, and among them, benzoyl peroxide is more preferable.

Examples of the azo compounds include 2,2'-azobis(isobutyronitrile), 2,2'-azobis(2,4-dimethylvaleronitrile), 4,4'-azobis(4-cyanovaleric acid), 1,1'-azobis(cyclohexane-1-carbonitrile), dimethyl-2,2'-azobis(isobutyrate), and 2,2'-azobis(2-amidinopropane)dihydrochloride.

Examples of the photopolymerization initiator include (bis)acylphosphine oxides, $\alpha$-diketones, and coumarins.

Examples of acylphosphine oxides among the (bis)acylphosphine oxides include 2,4,6-trimethylbenzoyl diphenylphosphine oxide, 2,6-dimethoxybenzoyl diphenylphosphine oxide, 2,6-dichlorobenzoyl diphenylphosphine oxide, 2,4,6-trimethylbenzoyl methoxyphenylphosphine oxide, 2,4,6-trimethylbenzoyl ethoxyphenylphosphine oxide, 2,3,5,6-tetramethylbenzoyl diphenylphosphine oxide, benzoyl di(2,6-dimethylphenyl)phosphonate, and salts thereof. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphineoxide, and salts thereof.

Among these (bis)acylphosphine oxides, 2,4,6-trimethylbenzoyl diphenylphosphine oxide, 2,4,6-trimethylbenzoyl methoxyphenylphosphine oxide, bis(2,4,6-trimethylbenzoyl)phenylphosphine oxide, and 2,4,6-trimethylbenzoyl phenylphosphine oxide sodium salt are preferable.

Examples of the $\alpha$-diketones include diacetyl, benzyl, camphorquinone, 2,3-pentadione, 2,3-octadione, 9,10-phenanthrenequinone, 4,4'-oxybenzyl, and acenaphthenequinone. Among them, camphorquinone is suitable.

Examples of the coumarins include compounds described in JP H9-3109 A and JP H10-245525 A, such as 3,3'-carbonylbis(7-diethylaminocoumarin), 3-(4-methoxybenzoyl)coumarin, 3-thenoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 7-methoxy-3-(p-nitrobenzoyl)coumarin, 3-(p-nitrobenzoyl)coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazole-2-ylidene)acetyl]coumarin, 3-[(1-methylnaphtho[1,2-d]thiazole-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazoyl)-7-(diethylamino)coumarin, 3-(2-benzothiazoyl)-7-(dibutylamino)coumarin, 3-(2-benzoimidazoyl)-7-(diethylamino)coumarin, 3-(2-benzothiazoyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrano[6, 7,8-ij]quinolizin-11-one, and 10-(2-benzothiazoyl)-2,3,6,7-tetrahydro-1,1,7,7-tetramethyl-1H,5H,11H-[1]benzopyrano[6,7,8-ij]quinolizin-11-one.

Among the above-described coumarin compounds, 3,3'-carbonylbis(7-diethylaminocoumarin) and 3,3'-carbonylbis(7-dibutylaminocoumarin) are suitable.

Among these photopolymerization initiators, at least one selected from the group consisting of (bis)acylphosphine oxides, $\alpha$-diketones, and coumarins that are widely used in dental curable compositions is preferably used.

When the photopolymerization initiator is used in combination with a polymerization accelerator if necessary, photopolymerization may be able to be more efficiently carried out in a shorter time.

Examples of polymerization accelerators suitable for the photopolymerization initiator mainly include tertiary amines, aldehydes, compounds having a thiol group, and sulfinic acids and/or salts thereof.

Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, n-butoxyethyl 4-(N,N-dimethylamino)benzoate, (2-methacryloyloxy)ethyl 4-(N,N-dimethylamino)benzoate, ethyl 4-(N,N-dimethylamino)benzoate, butyl 4-(N,N-dimethylamino)benzoate, N-methyldiethanolamine, 4-(N,N-dimethylamino)benzophenone, trimethylamine, triethylamine, N-methyldiethanolamine, N-ethyldiethanolamine, N-n-butyldiethanolamine, N-lauryldiethanolamine, triethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

Examples of the aldehydes include dimethylaminobenzaldehyde and terephthalaldehyde. Examples of the compounds having a thiol group include 2-mercaptobenzoxazole, decanethiol, 3-mercaptopropyltrimethoxysilane, and thiobenzoic acid.

Examples of the sulfinic acids and salts thereof include benzenesulfinic acid, sodium benzenesulfinate, potassium benzenesulfinate, calcium benzenesulfinate, lithium benzenesulfinate, p-toluenesulfinic acid, p-sodium toluenesulfinate, p-potassium toluenesulfinate, p-calcium toluenesulfinate, p-lithium toluenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, lithium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, and calcium 2,4,6-triisopropylbenzenesulfinate.

As the chemical polymerization initiator, redox polymerization initiators, such as organic peroxide and amine-based polymerization initiators; and organic peroxide, amine, and sulfinic acid (or salt thereof)-based polymerization initiators, are preferably used. When such a redox polymerization initiator is used, an oxidizing agent and a reducing agent need to be packaged separately and be mixed together immediately before use. Examples of the oxidizing agent for the redox polymerization initiator include organic peroxides. The organic peroxides as the oxidizing agent for the redox polymerization initiator are not particularly limited, and known organic peroxides can be used. Specific examples of the organic peroxides include the organic peroxides exemplified above for the thermal polymerization initiator.

Among these organic peroxides, the diacyl peroxides are preferably used from the viewpoint of the overall balance among safety, storage stability, and radical generation capability. Among the diacyl peroxides, benzoyl peroxide is more preferably used.

As the reducing agent for the redox polymerization initiator, aromatic tertiary amines having no electron withdrawing group in an aromatic ring are normally used. Examples of the aromatic tertiary amines having no electron withdrawing group in an aromatic ring include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, and N,N-bis(2-hydroxyethyl)-3,5-di-t-butylaniline.

The chemical polymerization initiator may be further used in combination with a polymerization accelerator if necessary. The polymerization accelerator for the chemical polymerization initiator may be selected for use from among polymerization accelerators commonly used in industry. Among them, a polymerization accelerator used for dentistry is preferably used. As the polymerization accelerator, one polymerization accelerator may be used alone, or two or more polymerization accelerators may be used in combination as appropriate. Specific examples of the polymerization accelerator for the chemical polymerization initiator include amines, sulfinic acids and salts thereof, copper compounds, and tin compounds.

The amines used as the polymerization accelerator for the chemical polymerization initiator are classified into aliphatic amines and aromatic amines having an electron withdrawing group in an aromatic ring. Examples of the aliphatic amines include: aliphatic primary amines such as n-butylamine, n-hexylamine, and n-octylamine; aliphatic secondary amines such as diisopropylamine, dibutylamine, and N-methylethanolamine; and aliphatic tertiary amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. Among them, the aliphatic tertiary amines are preferable from the viewpoint of curability and storage stability of the composition. Among the aliphatic tertiary amines, N-methyldiethanolamine and triethanolamine are more preferably used.

Examples of the aromatic tertiary amines having an electron withdrawing group in an aromatic ring that are used as the polymerization accelerator for the chemical polymerization initiator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-N,N-dimethylaminobenzoate, 4-(N,N-dimethylamino)benzophenone, and butyl 4-(N,N-dimethylamino)benzoate. Among them, at least one selected from the group consisting of N,N-di(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone is preferably used from the viewpoint that excellent curability can be imparted to the composition.

The sulfinic acid and salts thereof used as the polymerization accelerator include those exemplified above as the polymerization accelerator for the photopolymerization initiator. Sodium benzenesulfinate, sodium p-toluenesulfinate, and sodium 2,4,6-triisopropylbenzenesulfinate are preferable.

Examples of the copper compounds that are suitably used as the polymerization accelerator include copper acetylacetonate, copper(II) acetate, copper oleate, copper(II) chloride, and copper(II) bromide.

Examples of the tin compounds used as the polymerization accelerator include di-n-butyltin dimaleate, di-n-octyltin dimaleate, di-n-octyltin dilaurate, and di-n-butyltin dilaurate. Particularly suitable tin compounds are di-n-octyltin dilaurate and di-n-butyltin dilaurate.

Among them, for dental composite resins, a photopolymerization initiator is preferably used for the convenience of curing in an oral cavity, while for dental mill blanks, a thermal polymerization initiator is preferably used since the degree of polymerization is increased to improve the strength.

The content of the polymerization initiator (D) in the present invention is not particularly limited. However, from the viewpoint of the curability, etc., of the obtained composition, the content of the polymerization initiator (D) is preferably 0.001 to 30 parts by mass with respect to 100 parts by mass of the polymerizable monomer. When the content of the polymerization initiator (D) is 0.001 parts by mass or more with respect to 100 parts by mass of the polymerizable monomer, polymerization proceeds sufficiently and thus there is no risk of a decrease in mechanical strength. The content of the polymerization initiator (D) is more preferably 0.05 parts by mass or more and further preferably 0.1 parts by mass or more with respect to 100 parts by mass of the polymerizable monomer. Meanwhile, when the content of the polymerization initiator (D) is 30 parts by mass or less with respect to 100 parts by mass of the polymerizable monomer, sufficient mechanical strength can be achieved even if the polymerization performance of the polymerization initiator itself is low, and furthermore there is no risk of precipitation from the composition. The content of the polymerization initiator (D) is more preferably 20 parts by mass or less with respect to 100 parts by mass of the polymerizable monomer.

In addition to the above components, a pH adjuster, an ultraviolet absorber, an antioxidant, a coloring agent (pigment, dye), an antibacterial agent, an X-ray contrast agent, a thickener, a fluorescent agent, a crosslinking agent (metal ion-emitting component such as a polyvalent metal ion-emitting filler), etc., can be further added to the dental restorative curable composition of the present invention, depending on the purpose. As one embodiment, a dental restorative curable composition that does not contain a metal ion-emitting component such as a polyvalent metal ion-emitting filler is exemplified from the viewpoint of suppressing an increase in crosslink density, since an excessively high crosslink density causes increased brittleness.

As the pigment, known pigments that are used in dental composite resins are used without any limitations. The pigment may be any of an inorganic pigment and/or an organic pigment. Examples of the inorganic pigment include: chromates such as chromium yellow, zinc yellow, and barium yellow; ferrocyanides such as iron blue; sulfides such as vermilion, cadmium yellow, zinc sulfide, and cadmium red; sulfates such as barium sulfate, zinc sulfate, and strontium sulfate; oxides such as antimony white, zinc flower, titanium white, red oxide, black iron, and chromium oxide; hydroxides such as aluminum hydroxide; silicates such as calcium silicate and lapis lazuli; and carbons such as carbon black and graphite. Examples of the organic pigment include: nitrone-based pigments such as Naphthol Green B and Naphthol Green Y; nitro-based pigments such as Naphthol Yellow S and Lithol Fast Yellow 2G; insoluble azo-based pigments such as Permanent Red 4R, Brilliant Fast Scarlet, Hansa Yellow, and Benzidine Yellow; poorly soluble azo-based pigments such as Lithol Red, Lake Red C, and Lake Red D; soluble azo-based pigments such as Brilliant Carmine 6B, Permanent Red F5R, Pigment Scarlet 3B, and Bordeaux 10B; phthalocyanine-based pigments such as Phthalocyanine Blue, Phthalocyanine Green, and Sky Blue; basic dye-based pigments such as Rhodamine Lake, Malachite Green Lake,

and Methyl Violet Lake; and acidic dye-based pigments such as Peacock Blue Lake, Eosin Lake, and Quinoline Yellow Lake. One of these pigments may be used individually, or two or more of these pigments may be used in combination, and the pigment is selected as appropriate according to the intended color tone.

The content of the pigment in the dental restorative curable composition is adjusted as appropriate according to the desired color tone, and thus is not particularly limited. The content of the pigment is preferably 0.000001 parts by mass or more and more preferably 0.00001 parts by mass or more, and is preferably 5 parts by mass or less and more preferably 1 part by mass or less, with respect to 100 parts by mass of the dental restorative curable composition.

A method for producing the dental restorative curable composition of the present invention includes, for example, the following steps (1) and (2).

(1) Kneading Step

A kneading step is a step of performing a kneading operation in which polymerizable monomers such as the (meth)acrylic acid ester compound (A) and the (meth)acrylic acid ester compound (B) and the polymerization initiator (D) are put into a vessel of a kneading machine to prepare a polymerizable monomer-containing composition, then the inorganic filler (C) is further put into the vessel, and kneading is performed to produce a paste-like composition. In the kneading step, the kneading method is not particularly limited as long as the effects of the present invention are achieved, and a known method may be adopted. From the viewpoint of shortening the kneading time and preventing paste variation, it is preferable to perform the kneading while heating. The kneading temperature is preferably 40 to 60°C. If the kneading temperature is lower than 40°C, the kneading time shortening effect cannot be sufficiently achieved, and if the kneading temperature is higher than 60°C, polymerization curing and degradation of the composition may occur during the kneading. In addition, a vacuum defoaming treatment can also be performed during the kneading if necessary. The degree of vacuum in this case is not particularly limited. To efficiently remove air bubbles, the degree of vacuum is preferably 5 to 200 Torr.

(2) Defoaming Step

A defoaming step is a step of performing a defoaming operation in which after the paste-like composition is put into a vessel of a defoaming machine, defoaming is performed on the paste-like composition by extruding the paste out of the vessel under pressure while removing air bubbles inside the paste by decompression. The defoaming conditions are not particularly limited. To efficiently remove air bubbles, the degree of vacuum is preferably 5 to 200 Torr in order to suppress separation of the polymerizable monomer-containing composition, which contains the polymerizable monomers such as the (meth)acrylic acid ester compound (A) and the (meth)acrylic acid ester compound (B) and the polymerization initiator (D), and the inorganic filler (C). The decompression time is preferably 3 to 30 minutes. Also, the pressure at extrusion is preferably 0.5 to 5 MPa. The compression time is preferably 3 to 30 minutes. Moreover, a heat treatment can also be performed during the defoaming if necessary. The temperature in this case is not particularly limited. To efficiently remove air bubbles, the temperature is preferably 40 to 60°C.

A cured product of the dental restorative curable composition of the present invention has high mechanical strength and toughness and excellent water resistance and smoothness/gloss durability. Furthermore, the handling properties of the dental restorative curable composition are excellent, and air bubbles generated in the cured product can be reduced, resulting in excellent appearance. Therefore, the dental restorative curable composition of the present invention can be suitably used as a dental material. Specifically, the dental restorative curable composition of the present invention can be suitably used as a dental material (particularly, a dental composite resin) that can serve as a substitute for a part or the entirety of a natural tooth in the field of dentistry. Therefore, as one embodiment of the present invention, a dental composite resin comprising a dental restorative curable composition is exemplified. In addition, a cured product obtained by polymerizing and curing the dental restorative curable composition of the present invention can be suitably used as a dental mill blank that is a material to be cut and is used in a CAD/CAM system in which cutting is performed with a milling device for fabrication. Therefore, as another embodiment of the present invention, a dental mill blank comprising a cured product of the dental restorative curable composition is exemplified.

EXAMPLES

Hereinafter, the present invention will be specifically described by presenting Examples and Comparative Examples, but the present invention is not limited to the following Examples.

[(Meth)acrylic acid ester compound (A)]

PCIS-3: Synthesis Example 1
PCN-23: Synthesis Example 2
PCD-33: Synthesis Example 3
PARM: Synthesis Example 4
PSM: Synthesis Example 5
PCUMA: Synthesis Example 6

[(Meth)acrylic acid ester compound (B)]

D2.6E: 2,2-bis[4-methacryloyloxypolyethoxyphenyl]propane (average addition mole number of ethoxy group: 2.6) (manufactured by SHIN-NAKAMURA CHEMICAL Co., Ltd.)
UDMA: [2,2,4-trimethylhexamethylenebis(2-carbamoyloxyethyl)]dimethacrylate (manufactured by Kyoeisha Chemical Co., Ltd.)
NPG: neopentyl dimethacrylate (manufactured by Kyoeisha Chemical Co., Ltd.)
TEGDMA: triethylene glycol dimethacrylate (manufactured by SHIN-NAKAMURA CHEMICAL Co., Ltd.)

[Inorganic filler (C)]

Filler (F1): Production Example A
Filler (F2): Production Example B
Filler (F3): Production Example C
Filler (F5): Production Example D

[Organic filler]

Filler (F4): J-4PY (acrylic beads J-4PY, average primary particle diameter: 2.2 μm, manufactured by Negami Chemical Industrial Co., Ltd.)

[Polymerization initiator (D)]

CQ: camphorquinone (photopolymerization initiator) (manufactured by Tokyo Chemical Industry Co., Ltd.)
TPO: 2,4,6-trimethylbenzoyl diphenylphosphine oxide (photopolymerization initiator) (manufactured by Tokyo Chemical Industry Co., Ltd.)
THP: 1,1,3,3-tetramethylbutyl hydroperoxide (thermal polymerization initiator) (manufactured by NOF Corporation)
BPO: benzoyl peroxide (thermal polymerization initiator) (manufactured by NOF Corporation)

[Mono(meth)acrylic acid ester compound (E-1)]

POBA: m-phenoxybenzyl acrylate (manufactured by Kyoeisha Chemical Co., Ltd.)
[Mono(meth)acrylic acid ester compound (E-2)]
EPPA: ethoxylated-o-phenylphenol acrylate (manufactured by SHIN-NAKAMURA CHEMICAL Co., Ltd.)

[Surface treatment agent]

γ-MPS: γ-methacryloyloxypropyltrimethoxysilane (manufactured by Shin-Etsu Chemical Co., Ltd.)
11-MUS: 11-methacryloyloxydodecyltrimethoxysilane (manufactured by Shin-Etsu Chemical Co., Ltd.)

[Polymerization accelerator]

JJA: ethyl 4-(N,N-dimethylamino)benzoate

[Synthesis Example 1: Synthesis of PCIS-3]

80 g of a terminal hydroxy group-containing polycarbonate diol having an isosorbide skeleton "BENEBiOLH S0840H (molecular weight: 800, manufactured by Mitsubishi Chemical Corporation)", 40 g of triethylamine (molecular weight: 101.19), and 20 g of 4-dimethylaminopyridine (molecular weight: 122.17) were dissolved in methylene chloride, 24 g of methacryloyl chloride (molecular weight: 104.53) was added dropwise at 0°C, and then the mixture was stirred at room temperature for 24 hours. Then, the resulting organic layer was washed with distilled water, a 1M hydrochloric acid aqueous solution, and a saturated sodium carbonate aqueous solution in this order, and dried with magnesium sulfate. The dried solution was concentrated and then poured into methanol, and the resulting precipitate was collected by filtration and vacuum dried to obtain PCIS-3 (number average molecular weight: 936) which is a methacrylic acid ester compound having a polycarbonate structure in which the hydroxy residues at both ends derived from repeating units constituting a polymer structure are directly methacryloylated.

[Synthesis Example 2: Synthesis of PCN-23]

200 g of a terminal hydroxy group-containing polycarbonate diol having a neopentyl skeleton "BENEBiOLH NL2030B (molecular weight: 2,000, manufactured by Mitsubishi Chemical Corporation)", 40 g of triethylamine, and 20 g of 4-dimethylaminopyridine were dissolved in methylene chloride, 24 g of methacryloyl chloride was added dropwise at 0°C, and then the mixture was stirred at room temperature for 24 hours. Then, the resulting organic layer was washed with distilled water, a 1M hydrochloric acid aqueous solution, and a saturated sodium carbonate aqueous solution in this order, and dried with magnesium sulfate. The dried solution was concentrated and then poured into methanol, and the resulting precipitate was collected by filtration and vacuum dried to obtain PCN-23 (number average molecular weight: 2136) which is a methacrylic acid ester compound having a polycarbonate structure in which the hydroxy residues at both ends derived from repeating units constituting a polymer structure are directly methacryloylated.

[Synthesis Example 3: Synthesis of PCD-33]

300 g of a terminal hydroxy group-containing polycarbonate diol having a decyl skeleton "BENEBiOLH NL3010DB (molecular weight: 3,000, manufactured by Mitsubishi Chemical Corporation)", 40 g of triethylamine, and 20 g of 4-dimethylaminopyridine were dissolved in methylene chloride, 24 g of methacryloyl chloride was added dropwise at 0°C, and then the mixture was stirred at room temperature for 24 hours. Then, the resulting organic layer was washed with distilled water, a 1M hydrochloric acid aqueous solution, and a saturated sodium carbonate aqueous solution in this order, and dried with magnesium sulfate. The dried solution was concentrated and then poured into methanol, and the resulting precipitate was collected by filtration and vacuum dried to obtain PCD-33 (number average molecular weight: 3136) which is a methacrylic acid ester compound having a polycarbonate structure in which the hydroxy residues at both ends derived from repeating units constituting a polymer structure are directly methacryloylated.

[Synthesis Example 4: Synthesis of PARM]

85 g of bisphenol A (molecular weight: 228.29) and 30 g of sodium hydroxide (molecular weight: 40.00) were dissolved in distilled water, 10 mg of tetrabutylammonium bromide (molecular weight: 322.37) was further added, and the mixture was stirred at room temperature for 10 minutes. Then, a methylene chloride solution containing 30 g of terephthaloyl chloride (molecular weight: 203.02) was added dropwise, and the mixture was stirred vigorously at room temperature for 1 hour. Then, the resulting organic layer was washed with distilled water and dried with magnesium sulfate. The dried solution was concentrated and then poured into a methanol/water (15/1) mixed solution, and the resulting precipitate was collected by filtration and vacuum-dried to obtain a polyarylate "PAROH (molecular weight: 900)" having hydroxy groups on both ends. 90 g of the obtained PAROH, 40 g of triethylamine, and 20 g of 4-dimethylaminopyridine were dissolved in methylene chloride, 24 g of methacryloyl chloride was added dropwise at 0°C, and then the mixture was stirred at room temperature for 24 hours. Then, the resulting organic layer was washed with distilled water, a 1M hydrochloric acid aqueous solution, and a saturated sodium carbonate aqueous solution in this order, and dried with magnesium sulfate. The dried solution was concentrated and then poured into methanol, and the resulting precipitate was collected by filtration and vacuum dried to obtain PARM (number average molecular weight: 1036) which is a methacrylic acid ester compound having a polyarylate structure in which the hydroxy residues at both ends derived from repeating units constituting a polymer structure are directly methacryloylated.

[Synthesis Example 5: Synthesis of PSM]

85 g of bisphenol A and 30 g of sodium hydroxide were dissolved in toluene, 40 g of N-methylpyrrolidone (molecular weight: 99.13) was further added, and the mixture was stirred at 140°C for 2 hours. Then, 43 g of 4,4'-dichlorodiphenylsulfone (molecular weight: 287.15) was added, and the mixture was stirred at 160°C for 12 hours. After cooling to room temperature, 24 g of methacryloyl chloride was added dropwise, and the mixture was stirred at 60°C for 10 hours. The precipitate resulting from the addition of methylene chloride was then removed by filtration, and the filtrate was washed with a 1 wt% oxalic acid aqueous solution and dried with magnesium sulfate. The dried solution was concentrated and then poured into methanol, and the resulting precipitate was collected by filtration and vacuum dried to obtain PSM (number average molecular weight: 1232) which is a methacrylic acid ester compound having a polysulfone structure in which the hydroxy residues at both ends derived from repeating units constituting a polymer structure are directly methacryloylated.

[Synthesis Example 6: Synthesis of PCUMA]

80 g of a terminal hydroxy group-containing polycarbonate diol having an isosorbide skeleton "BENEBiOLH S0840H" and 25 mg of dibutyltin dilaurate (molecular weight: 631.57) were dissolved in THF, 50 g of Karenz MOI-EG (molecular weight: 199.20, manufactured by Showa Denko K.K.) was added dropwise at room temperature, and then the mixture was stirred at 40°C for 24 hours. After the reaction, the solution was concentrated, methylene chloride was added, and then the solution was washed with a 1M hydrochloric acid aqueous solution and dried with magnesium sulfate. The dried solution was concentrated and then poured into methanol, and the resulting precipitate was collected by filtration and vacuum dried to obtain PCUMA (number average molecular weight: 1200) which is a methacrylic acid ester compound having a polycarbonate structure in which an alkylene group, an ether bond, and a urethane bond exist between each of the hydroxy residues at both ends derived from repeating units constituting a polymer structure and each methacryloyl group.

[Measurement of number average molecular weight]

The number average molecular weight (Mn) of the (meth)acrylic acid ester compound (A) was measured by gel permeation chromatography (GPC) as follows.

First, the compound synthesized in each of Synthesis Examples 1 to 6 above was dissolved in tetrahydrofuran (THF, containing 0.5 mg/mL of BHT) at room temperature such that the concentration thereof was 0.5 mg/mL. The obtained solution was then filtered through a solvent-resistant membrane filter "Myshoridisk" (manufactured by Tosoh Corporation) having a pore diameter of 0.2 μm to obtain a sample solution. The sample solution was adjusted such that the concentration of a component soluble in THF was 0.8 mass%. Measurement was performed under the following conditions using this sample solution.

Apparatus: high-speed GPC apparatus "HLC-8220GPC" [manufactured by Tosoh Corporation]
Column: LF-604 × 2 [manufactured by Showa Denko K.K.]
Eluent: THF
Flow rate: 0.6 mL/min
Oven temperature: 40°C
Sample injection volume: 0.020 mL

For calculating the molecular weight of each sample, a calibration curve was created by using 0.2 mL of a solution obtained by dissolving each of standard polystyrene resins (e.g., "TSK Standard Polystyrene F-850, F-450, F-288, F-128, F-80, F-40, F-20, F-10, F-4, F-2, F-1, A-5000, A-2500, A-1000, and A-500", manufactured by Tosoh Corporation) in THF (containing 0.5 mg/mL of BHT) such that the concentration thereof was 0.5 mg/mL. As the calibration curve, a cubic expression obtained through approximation by the least-squares method was used.

The definition of the number average molecular weight (Mn) is described in "Fundamentals of Polymer Chemistry (Kobunshi Kagaku No Kiso)" (edited by The Society of Polymer Science, Japan, Tokyo Kagaku Doujin, 1978), and the number average molecular weight (Mn) can be calculated from a molecular weight distribution curve by GPC. In the case of an extremely low molecular weight, an elution peak of a sample may overlap with a solvent-derived ghost and become indistinct (under the above conditions, the molecular weight is approximately 400 g/mol or less), and in such a case, an average molecular weight is obtained by excluding components having a molecular weight of 400 g/mol or less from the calculation.

[Production Example 1: Production of polymerizable monomer-containing composition (M1)]

A polymerizable monomer-containing composition (M1) was prepared by dissolving 0.2 parts by mass of CQ as a photopolymerization initiator, 0.25 parts by mass of TPO, and 0.3 parts by mass of JJA as a polymerization accelerator in 80 parts by mass of D2.6E and 20 parts by mass of PCIS-3.

[Production Example 2: Production of polymerizable monomer-containing composition (M2)]

A polymerizable monomer-containing composition (M2) was prepared by dissolving 0.2 parts by mass of CQ as a photopolymerization initiator, 0.25 parts by mass of TPO, and 0.3 parts by mass of JJA as a polymerization accelerator in 80 parts by mass of D2.6E and 20 parts by mass of PCN-23.

[Production Example 3: Production of polymerizable monomer-containing composition (M3)]

A polymerizable monomer-containing composition (M3) was prepared by dissolving 0.2 parts by mass of CQ as a photopolymerization initiator, 0.25 parts by mass of TPO, and 0.3 parts by mass of JJA as a polymerization accelerator in 80 parts by mass of D2.6E and 20 parts by mass of PCD-33.

[Production Example 4: Production of polymerizable monomer-containing composition (M4)]

A polymerizable monomer-containing composition (M4) was prepared by dissolving 0.2 parts by mass of CQ as a photopolymerization initiator, 0.25 parts by mass of TPO, and 0.3 parts by mass of JJA as a polymerization accelerator in 80 parts by mass of D2.6E and 20 parts by mass of PARM.

[Production Example 5: Production of polymerizable monomer-containing composition (M5)]

A polymerizable monomer-containing composition (M5) was prepared by dissolving 0.2 parts by mass of CQ as a photopolymerization initiator, 0.25 parts by mass of TPO, and 0.3 parts by mass of JJA as a polymerization accelerator in 80 parts by mass of D2.6E and 20 parts by mass of PSM.

[Production Example 6: Production of polymerizable monomer-containing composition (M6)]

A polymerizable monomer-containing composition (M6) was prepared by dissolving 0.2 parts by mass of CQ as a photopolymerization initiator, 0.25 parts by mass of TPO, and 0.3 parts by mass of JJA as a polymerization accelerator in 80 parts by mass of UDMA and 20 parts by mass of PCIS-3.

[Production Example 7: Production of polymerizable monomer-containing composition (M7)]

A polymerizable monomer-containing composition (M7) was prepared by dissolving 0.2 parts by mass of CQ as a photopolymerization initiator, 0.25 parts by mass of TPO, and 0.3 parts by mass of JJA as a polymerization accelerator in 70 parts by mass of D2.6E, 10 parts by mass of NPG, and 20 parts by mass of PCIS-3.

[Production Example 8: Production of polymerizable monomer-containing composition (M8)]

A polymerizable monomer-containing composition (M8) was prepared by dissolving 0.2 parts by mass of CQ as a photopolymerization initiator, 0.25 parts by mass of TPO, and 0.3 parts by mass of JJA as a polymerization accelerator in 70 parts by mass of D2.6E, 20 parts by mass of PCIS-3, and 10 parts by mass of POBA.

[Production Example 9: Production of polymerizable monomer-containing composition (M9)]

A polymerizable monomer-containing composition (M9) was prepared by dissolving 0.2 parts by mass of CQ as a photopolymerization initiator, 0.25 parts by mass of TPO, and 0.3 parts by mass of JJA as a polymerization accelerator in 70 parts by mass of D2.6E, 20 parts by mass of PCIS-3, and 10 parts by mass of EPPA.

[Production Example 10: Production of polymerizable monomer-containing composition (M10)]

A polymerizable monomer-containing composition (M10) was prepared by dissolving 0.5 parts by mass of THP as a thermal polymerization initiator in 80 parts by mass of D2.6E and 20 parts by mass of PCIS-3.

[Production Example 11: Production of polymerizable monomer-containing composition (M11)]

A polymerizable monomer-containing composition (M11) was prepared by dissolving 0.2 parts by mass of CQ as a photopolymerization initiator, 0.25 parts by mass of TPO, and 0.3 parts by mass of JJA as a polymerization accelerator in 80 parts by mass of D2.6E and 20 parts by mass of PCUMA.

[Production Example 12: Production of polymerizable monomer-containing composition (M12)]

A polymerizable monomer-containing composition (M12) was prepared by dissolving 0.2 parts by mass of CQ as a photopolymerization initiator, 0.25 parts by mass of TPO, and 0.3 parts by mass of JJA as a polymerization accelerator in 80 parts by mass of D2.6E and 20 parts by mass of NPG.

[Production Example 13: Production of polymerizable monomer-containing composition (M13)]

A polymerizable monomer-containing composition (M13) was prepared by dissolving 0.5 parts by mass of THP as a thermal polymerization initiator in 80 parts by mass of D2.6E and 20 parts by mass of PCUMA.

[Production Example 14: Production of polymerizable monomer-containing composition (M14)]

A polymerizable monomer-containing composition (M14) was prepared by dissolving 1.5 parts by mass of BPO as a thermal polymerization initiator in 70 parts by mass of UDMA and 30 parts by mass of TEGDMA.

The composition of each polymerizable monomer-containing composition is shown in Table 1 below.

[Table 1]

| Component (parts by mass) | Polymerizable monomer-containing composition | | | | | | | | | | | | | |
|---|---|---|---|---|---|---|---|---|---|---|---|---|---|---|
| | M1 | M2 | M3 | M4 | M5 | M6 | M7 | M8 | M9 | M10 | M11 | M12 | M13 | M14 |
| (Meth)acrylic acid ester compound (A) | | | | | | | | | | | | | | |
| PCIS-3 | 20 | | | | | 20 | 20 | 20 | 20 | 20 | | | | |
| PCN-23 | | 20 | | | | | | | | | | | | |
| PCD-33 | | | 20 | | | | | | | | | | | |
| PARM | | | | 20 | | | | | | | | | | |
| PSM | | | | | 20 | | | | | | | | | |
| (Meth)acrylic acid ester compound not included in (A), (B), and (E) | | | | | | | | | | | | | | |
| PCUMA | | | | | | | | | | | 20 | | 20 | |
| (Meth)acrylic acid ester compound (B) (excluding (meth)acrylic acid ester compound (A)) | | | | | | | | | | | | | | |
| D2.6E | 80 | 80 | 80 | 80 | 80 | | 70 | 70 | 70 | 80 | 80 | 80 | 80 | |
| UDMA | | | | | | 80 | | | | | | | | 70 |
| NPG | | | | | | | 10 | | | | | 20 | | |
| TEGDMA | | | | | | | | | | | | | | 30 |
| Polymerization initiator (D) | | | | | | | | | | | | | | |
| CQ | 0.2 | 0.2 | 0.2 | 0.2 | 0.2 | 0.2 | 0.2 | 0.2 | 0.2 | | 0.2 | 0.2 | | |
| TPO | 0.25 | 0.25 | 0.25 | 0.25 | 0.25 | 0.25 | 0.25 | 0.25 | 0.25 | | 0.25 | 0.25 | | |
| THP | | | | | | | | | | 0.5 | | | 0.5 | |
| BPO | | | | | | | | | | | | | | 1.5 |
| Polymerization accelerator | | | | | | | | | | | | | | |
| JJA | 0.3 | 0.3 | 0.3 | 0.3 | 0.3 | 0.3 | 0.3 | 0.3 | 0.3 | | 0.3 | 0.3 | | |
| Mono(meth)acrylic acid ester compounds (E-1) and (E-2) | | | | | | | | | | | | | | |
| POBA | | | | | | | | 10 | | | | | | |

(continued)

| Component (parts by mass) | Polymerizable monomer-containing composition | | | | | | | | | | | | | |
|---|---|---|---|---|---|---|---|---|---|---|---|---|---|---|
| | M1 | M2 | M3 | M4 | M5 | M6 | M7 | M8 | M9 | M10 | M11 | M12 | M13 | M14 |
| EPPA | | | | | | | | | 10 | | | | | |

[Production Example A: Production of filler (F1)]

In 300 parts by mass of ethanol, 100 parts by mass of NF180 (barium glass (average primary particle diameter: 0.180 μm, manufactured by SCHOTT)) was dispersed, and 7 parts by mass of γ-MPS, 0.15 parts by mass of acetic acid, and 5 parts by mass of water were added. The mixture was stirred at room temperature for 2 hours. The solvent was distilled off under reduced pressure, and a surface treatment was further performed by drying at 90°C for 3 hours to obtain a filler (F1). F1 is an inorganic filler (C).

[Production Example B: Production of filler (F2)]

In 300 parts by mass of ethanol, a mixture of 80 parts by mass of UF2.0 (barium glass (average primary particle diameter: 2.0 μm, manufactured by SCHOTT)) and 20 parts by mass of NF180 (barium glass (average primary particle diameter: 0.180 μm, manufactured by SCHOTT)) was dispersed, and 2.25 parts by mass of γ-MPS, 0.15 parts by mass of acetic acid, and 5 parts by mass of water were added. The mixture was stirred at room temperature for 2 hours. The solvent was distilled off under reduced pressure, and a surface treatment was further performed by drying at 90°C for 3 hours to obtain a filler (F2). F2 is an inorganic filler (C).

[Production Example C: Production of filler (F3)]

In 300 parts by mass of ethanol, a mixture of 90 parts by mass of UF0.7 (barium glass (average primary particle diameter: 0.7 μm, manufactured by SCHOTT)) and 10 parts by mass of Ar130 (particulate silica (average primary particle diameter: 0.016 μm, manufactured by NIPPON AEROSIL CO., LTD.)) was dispersed, 4 parts by mass of 11-MUS, 0.15 parts by mass of acetic acid, and 5 parts by mass of water were added. The mixture was stirred at room temperature for 2 hours. The solvent was distilled off under reduced pressure, and a surface treatment was further performed by drying at 90°C for 3 hours to obtain a filler (F3). F3 is an inorganic filler (C).

[Production Example D: Production of filler (F5)]

In 300 parts by mass of ethanol, 100 parts by mass of Ox50 (particulate silica (average primary particle diameter: 0.04 μm, manufactured by NIPPON AEROSIL CO., LTD.)) was dispersed, and 7 parts by mass of γ-MPS, 0.15 parts by mass of acetic acid, and 5 parts by mass of water were added. The mixture was stirred at room temperature for 2 hours. The solvent was distilled off under reduced pressure, and a surface treatment was further performed by drying at 90°C for 3 hours to obtain a filler (F5). F5 is an inorganic filler (C).

The compositions of the fillers (F1 to F5) are shown in Table 2 below.

[Table 2]

| Component (parts by mass) | Filler | | | | |
|---|---|---|---|---|---|
| | F1 | F2 | F3 | F4 | F5 |
| Inorganic filler | | | | | |
| UF2.0 | | 80 | | | |
| UF0.7 | | | 90 | | |
| NF180 | 100 | 20 | | | |
| Ar130 | | | 10 | | |
| Ox50 | | | | | 100 |
| Organic filler | | | | | |
| J-4PY | | | | 100 | |
| Surface treatment agent | | | | | |
| γ-MPS | 7 | 2.25 | | | 7 |
| 11-MUS | | | 4 | | |

[Initial mechanical strength and toughness (3-point flexural test)]

As for the dental composite resin, the produced dental restorative curable composition of each Example and Comparative Example was defoamed in vacuum, and then was charged into a stainless steel mold (dimensions: 2 mm × 2 mm × 25 mm). The dental restorative curable composition was pressed between glass slides from above and below, and light was applied to both sides, using a dental visible light irradiator (PenCure 2000, manufactured by J. Morita Corp.), such that the light was applied at 5 points on each side, 10 seconds at each point, thereby curing the dental restorative curable composition. Five cured products were produced as samples for each Example and Comparative Example. After the cured product samples were taken out from the mold, the flexural strength and the fracture energy of each cured product sample were measured under the conditions of a distance of 20 mm between fulcrums and a crosshead speed of 1 mm/min, using a universal testing machine (trade name "AG-I 100 kN", manufactured by SHIMADZU CORPORATION). From the measured values of the respective samples, average values were calculated, and used as flexural strength (3-point flexural strength) and fracture energy. As for the dental mill blank, measurement was performed in the same manner as the above measurement on the samples of the dental composite resin, except that a test piece (1.2 mm × 4 mm × 15 mm) was prepared from a produced dental mill blank using a diamond cutter, the distance between fulcrums when using the universal testing machine was set to 12 mm, and ten cured products were produced as samples for each Example and Comparative Example. As for the flexural strength of the dental composite resin, 150 MPa or more is determined to be good, and 180 MPa or more is better. As for the fracture energy of the dental composite resin, 13 mJ or more is determined to be good, and 15 mJ or more is better. As for the flexural strength of the dental mill blank, 200 MPa or more is determined to be good, and 240 MPa or more is better. As for the fracture energy of the dental mill blank, 30 mJ or more is determined to be good, and 32 mJ or more is better. For the mechanical strength and the toughness, both the flexural strength and the fracture energy preferably satisfy the above ranges.

[Water resistance (3-point flexural test)]

As for the dental composite resin, the produced cured product samples of the dental restorative curable composition according to each Example and Comparative Example were immersed in water at 37°C for 30 days, and then the flexural strength and the fracture energy thereof were measured in the same manner as the initial mechanical strength (n=5). As for the dental mill blank, measurement was performed in the same manner as in that for the dental composite resin except n=10. The rates of change (rate of decrease) in flexural strength and fracture energy after immersion in water at 37°C for 30 days with respect to the initial flexural strength and fracture energy were calculated using the following equations. If the rate of decrease is 10% or less, the water resistance is excellent, and if the rate of decrease is 5% or less, the water resistance is further excellent.

Rate of decrease in flexural strength due to immersion (%) = [{initial flexural strength (MPa) - flexural strength after immersion in water (MPa)} / initial flexural strength (MPa)] × 100

Rate of decrease in fracture energy due to immersion (%) = [{initial fracture energy (mJ) - fracture energy after immersion in water (mJ)} / initial fracture energy (mJ)] × 100

[Smoothness/gloss durability]

As for the dental composite resin, the produced dental restorative curable composition of each Example and Comparative Example was defoamed in vacuum, and then was charged into a stainless steel mold (dimensions: 1.5 mm × 15 mm × 15 mm). The dental restorative curable composition was pressed between glass slides from top and bottom, and light was applied to both sides, using an LED curing unit for dental technique (α-Light V, manufactured by J. Morita Corp.), such that the light was applied to each side for 90 seconds, thereby curing the dental restorative curable composition. As for the dental mill blank, a test piece (1.5 mm × 15 mm × 15 mm) was prepared from a produced dental mill blank using a diamond cutter. The obtained cured product and the test piece thereof were polished under dry conditions with #1000 abrasive paper, #2000 abrasive paper, and #3000 abrasive paper in this order, and finally with a lapping film. For the test piece, the glossinesses before and after a toothbrush abrasion test (toothbrush: "Between Lion" (hardness: normal; manufactured by Lion Corporation), toothpaste: "Dentor Clear MAX" (manufactured by Lion Corporation), load: 250 g, test solution: distilled water/toothpaste = 90/10 (mass%, 50 mL), abrasion frequency: 40,000 times) were determined by a gloss meter (VG-2000, NIPPON DENSHOKU INDUSTRIES CO., LTD.) (n=2). As the glossiness, a 60° mirror surface gloss (Gs (60°)) was measured. A glossiness retention rate (%) was calculated by dividing the obtained glossiness after the toothbrush abrasion test by the glossiness before the test, and used for evaluation of smoothness/gloss durability. The glossiness retention rate is preferably 85% or more, more preferably 90% or more, and further preferably 93% or more.

[Paste handling properties (adhesion force)]

The produced dental restorative curable composition of each Example and Comparative Example was defoamed in vacuum, and then was charged into a syringe. The paste was allowed to stand at 25°C for 24 hours to prepare a sample for an adhesion force test. The paste was extruded from the syringe and charged into a cup having a capacity of a bottom face diameter of 11 mm, a top face diameter of 13 mm, and a height of 8 mm. A jig having a stainless steel cylinder with 10 mm in diameter × 5 mm at an end thereof was attached to a compact table-top tester (EZ Test, manufactured by SHIMADZU CORPORATION). The bottom surface of the stainless steel cylinder on the cylindrical jig was gently brought into contact with the charged paste, and then the maximum stress when the jig was lifted at a crosshead speed of 50 mm/min was measured as an adhesion force to a stainless steel plate at 25°C (n=2). An average value was calculated, and used for evaluation of paste handling properties. As for the evaluation of paste handling properties, the adhesion force is preferably 1.5 N or less and more preferably 1.0 N or less. On the other hand, if the adhesion force is 2.0 N or more, the paste handling properties when filling using a dental instrument is determined to be low.

[Air bubble generation rate in cured product]

Each produced dental mill blank was evaluated for the presence or absence of air bubbles in the interior and on the appearance of the cured product, using a desktop microfocus X-ray CT system (inspeXio SMX-90CT manufactured by SHIMADZU CORPORATION) (n=10). As for the evaluation of an air bubble generation rate in the cured product, the number of cured products with air bubbles is preferably 1 or less and more preferably 0. On the other hand, if the number of cured products with air bubbles is 3 or more, it is determined that the quality is poor in view of decrease in mechanical strength, deterioration in appearance, etc.

[Examples 1 to 12, Comparative Examples 1 to 3] (dental composite resin)

The polymerizable monomer-containing compositions (M1 to M9, M11, M12) and the fillers (F1 to F4) obtained in the Production Examples above were used, to be mixed and kneaded at the composition ratios shown in Table 3 below for uniformization, and then were defoamed in vacuum to prepare paste-like dental restorative curable compositions of Examples 1 to 12 and Comparative Examples 1 to 3. The prepared dental restorative curable compositions were tested. The results are shown in Table 3 below.

[Table 3]

| | | Example | | | | | | | |
|---|---|---|---|---|---|---|---|---|---|
| | | 1 | 2 | 3 | 4 | 5 | 6 | 7 | 8 |
| Filler | Type | (F1) | (F1) | (F1) | (F1) | (F1) | (F1) | (F1) | (F1) |
| | parts by mass | 75 | 75 | 75 | 75 | 75 | 75 | 75 | 75 |
| Polymerizable monomer-containinq composition | Type | (M1) | (M2) | (M3) | (M4) | (M5) | (M6) | (M7) | (M8) |
| | parts by mass | 25 | 25 | 25 | 25 | 25 | 25 | 25 | 25 |
| Various tests<br>Flexural strength (initial) | MPa | 187 | 174 | 162 | 183 | 181 | 179 | 176 | 184 |

(continued)

| | | Example | | | | | | | |
|---|---|---|---|---|---|---|---|---|---|
| | | 1 | 2 | 3 | 4 | 5 | 6 | 7 | 8 |
| Fracture energy (initial) | mJ | 17.2 | 18.0 | 19.5 | 16.6 | 16.3 | 18.8 | 17.4 | 18.1 |
| Flexural strength (after immersion in water at 37°C for 30 days) | MPa | 173 | 163 | 151 | 170 | 169 | 163 | 170 | 180 |
| Fracture energy (after immersion in water at 37°C for 30 days) | mJ | 15.9 | 16.8 | 18.1 | 15.4 | 15.2 | 17.5 | 16.4 | 17.5 |
| Rate of decrease in flexural strength due to immersion | % | 7.5 | 6.3 | 6.8 | 7.1 | 6.6 | 8.9 | 3.4 | 2.2 |
| Rate of decrease in fracture energy due to immersion | % | 7.6 | 6.7 | 7.2 | 7.2 | 6.7 | 6.9 | 5.7 | 3.3 |
| Smoothness/gloss durability | % | 94 | 91 | 90 | 93 | 94 | 92 | 92 | 94 |
| Paste handling properties (adhesion force) | N | 1.2 | 1.3 | 1.5 | 1.1 | 1.1 | 1.4 | 1.1 | 1.0 |

| | | Example | | | | Comparative Example | | |
|---|---|---|---|---|---|---|---|---|
| | | 9 | 10 | 11 | 12 | 1 | 2 | 3 |
| Filler | Type | (F1) | (F2) | (F3) | (F1) | (F1) | (F1) | (F4) |
| | parts by mass | 75 | 78 | 78 | 40 | 75 | 75 | 75 |
| Polymerizable monomer-containing composition | Type | (M9) | (M1) | (M1) | (M1) | (M11) | (M12) | (M1) |
| | parts by mass | 25 | 22 | 22 | 60 | 25 | 25 | 35 |
| Various tests | | | | | | | | |
| Flexural strength (initial) | MPa | 182 | 214 | 193 | 176 | 140 | 137 | 113 |
| Fracture energy (initial) | mJ | 17.9 | 15.1 | 17.8 | 19.7 | 16.3 | 8.4 | 17.4 |
| Flexural strength (after immersion in water at 37°C for 30 days) | MPa | 177 | 204 | 189 | 160 | 76 | 118 | 97 |
| Fracture energy (after immersion in water at 37°C for 30 days) | mJ | 17.2 | 14.3 | 16.9 | 18.0 | 11.2 | 7.1 | 14.9 |
| Rate of decrease in flexural strength due to immersion | % | 2.7 | 4.7 | 2.1 | 9.1 | 45.7 | 13.9 | 14.2 |

(continued)

| | | Example | | | | Comparative Example | | |
|---|---|---|---|---|---|---|---|---|
| | | 9 | 10 | 11 | 12 | 1 | 2 | 3 |
| Rate of decrease in fracture energy due to immersion | % | 3.9 | 5.3 | 5.1 | 8.6 | 31.3 | 15.5 | 14.4 |
| Smoothness/gloss durability | % | 94 | 90 | 95 | 86 | 77 | 81 | 32 |
| Paste handling properties (adhesion force) | N | 1.0 | 0.7 | 0.9 | 1.8 | 2.4 | 2.9 | 8.7 |

As shown in Table 3, the pastes obtained in Examples 1 to 12 were all good in handling properties. It was also found that the cured products thereof had high flexural strength and fracture energy, and had excellent water resistance with no strength decrease due to immersion in water at 37°C assuming an oral cavity. It was further found that the cured products had excellent smoothness/gloss durability with no decrease in glossiness due to abrasion by a toothbrush or the like. On the other hand, the cured product obtained in Comparative Example 1 did not have bad initial fracture energy, but had significantly low water resistance. In Comparative Example 1, it was considered that the density of the polymer structure was relatively low due to the structure, of PCUMA included in the compound disclosed in Patent Literature 1, in which an alkylene group and a urethane bond exist between each of the hydroxy residues at both ends derived from repeating units constituting the polymer structure and each methacryloyl group, and water was absorbed due to the presence of the urethane bond, resulting in lower water resistance. In addition, the cured product obtained in Comparative Example 2 had low water resistance and very low fracture energy. Furthermore, the cured product obtained in Comparative Example 3 had low mechanical strength, and the paste handling properties and the smoothness/gloss durability of the cured product thereof were very poor.

[Examples 13-15, Comparative Examples 4 and 5] (dental mill blank)

The polymerizable monomers (M10, M13, M14) and the fillers (F1 to F3, F5) obtained in the Production Examples above were used, to be mixed and kneaded at the composition ratios shown in Table 4 below for uniformization, and then were defoamed in vacuum to prepare paste-like dental restorative curable compositions of Examples 13 to 15 and Comparative Examples 4 and 5. Next, the dental restorative curable compositions were each poured into a rectangular mold of 20 mm × 30 mm × 60 mm and heated at 50°C for 1 hour. Then, a heat treatment was performed under pressure of 1 MPa at 150°C for 1 hour to obtain a cured product as a dental mill blank. The obtained cured products were tested. The results are shown in Table 4 below.

[Table 4]

| | | Example | | | Comparative Example | |
|---|---|---|---|---|---|---|
| | | 13 | 14 | 15 | 4 | 5 |
| Filler | Type parts by mass | (F1) | (F2) | (F3) | (F1) | (F5) |
| | | 75 | 80 | 78 | 75 | 60 |
| Polymerizable monomer-containinq composition | Type parts by mass | (M10) | (M10) | (M10) | (M13) | (M14) |
| | | 25 | 20 | 22 | 25 | 40 |
| Various tests | | | | | | |
| Flexural strength (initial) | MPa | 273 | 328 | 297 | 238 | 206 |
| Fracture energy (initial) | mJ | 31.0 | 33.6 | 32.9 | 26.8 | 13.4 |

(continued)

| | | Example | | | Comparative Example | |
|---|---|---|---|---|---|---|
| | | 13 | 14 | 15 | 4 | 5 |
| Flexural strength (after immersion in water at 37°C for 30 days) | MPa | 264 | 319 | 291 | 167 | 179 |
| Fracture energy (after immersion in water at 37°C for 30 days) | mJ | 30.1 | 32.8 | 32.3 | 18.2 | 11.8 |
| Rate of decrease in flexural strength due to immersion | % | 3.3 | 2.7 | 2.0 | 29.8 | 13.1 |
| Rate of decrease in fracture energy due to immersion | % | 2.9 | 2.4 | 1.8 | 32.1 | 11.9 |
| Smoothness/gloss durability | % | 93 | 90 | 93 | 82 | 73 |
| Air bubble generation rate in cured product | Piece | 0 | 0 | 0 | 6 | 3*1 |
| * 1 Number of cracks generated during production of polymerizable monomer impregnated molded body, etc. | | | | | | |

The cured products obtained in Examples 13 to 15 were all free from air bubbles. It was also found that the cured products obtained in Examples 13 to 15 had high flexural strength and fracture energy, and had excellent water resistance with no strength decrease due to immersion in water at 37°C assuming an oral cavity. It was further found that the cured products obtained in Examples 13 to 15 had excellent smoothness/gloss durability with no decrease in glossiness due to abrasion by a toothbrush or the like. On the other hand, in the cured product obtained in Comparative Example 4, some air bubbles were observed, and the water resistance was significantly low. In addition, in the cured product obtained in Comparative Example 5, cracks occurred during production of a polymerizable monomer impregnated molded body, etc., and the flexural strength and the fracture energy were very low.

From the above results, it was found that the dental restorative curable composition of the present invention has excellent paste handling properties, and the cured product thereof has high mechanical strength and toughness and excellent water resistance and smoothness/gloss durability.

INDUSTRIAL APPLICABILITY

The dental restorative curable composition of the present invention has excellent paste handling properties, and the cured product thereof has high mechanical strength and toughness and excellent water resistance and smoothness/gloss durability. That is, the dental restorative curable composition of the present invention is excellent in handling, has sufficient mechanical strength to serve as substitute for natural teeth, and is suitably used as a material for restoring lost parts and caries of teeth, in particular as a dental composite resin. Moreover, the cured product thereof is suitably used as a dental mill blank.

## Claims

**1.** A dental restorative curable composition comprising:

a (meth)acrylic acid ester compound (A) which has at least one polymer structure selected from the group consisting of a polycarbonate, a polyarylate, and an aromatic polysulfone and in which terminal hydroxy residues derived from repeating units constituting the polymer structure are directly (meth)acryloylated;
a (meth)acrylic acid ester compound (B) having two or more (meth)acryloyloxy groups (excluding the (meth)acrylic acid ester compound (A));
an inorganic filler (C) having an average primary particle diameter of 0.01 to 5 μm; and
a polymerization initiator (D).

**2.** The dental restorative curable composition according to claim 1, wherein the polymer structure has a ring structure.

3. The dental restorative curable composition according to claim 1 or 2, wherein the polymer structure comprises three or more functional groups that are each independently at least one functional group selected from the group consisting of carbonate groups, ester groups, and sulfonyl groups.

4. The dental restorative curable composition according to any one of claims 1 to 3, wherein the (meth)acrylic acid ester compound (A) has a number average molecular weight of 300 to 5,000.

5. The dental restorative curable composition according to any one of claims 1 to 4, wherein a content of the (meth)acrylic acid ester compound (A) is 1 to 40 mass% in a total amount of a polymerizable monomer.

6. The dental restorative curable composition according to any one of claims 1 to 5, further comprising at least one compound selected from the group consisting of a mono(meth)acrylic acid ester compound (E-1) represented by the following general formula (I) and a mono(meth)acrylic acid ester compound (E-2) represented by the following general formula (II),

[Chem. 1]

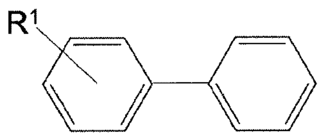

(I)

[Chem. 2]

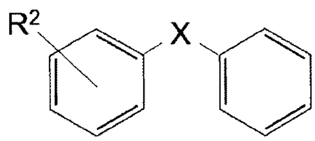

(II)

where $R^1$ and $R^2$ are each independently a group represented by the following general formula (i) or a group represented by the following general formula (ii), and X is a divalent hydrocarbon group having 1 to 6 carbon atoms, or an oxygen atom,

[Chem. 3]

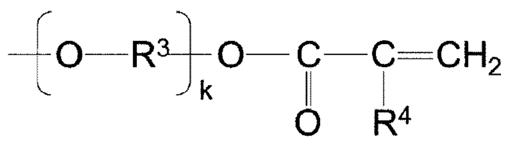

( i )

[Chem. 4]

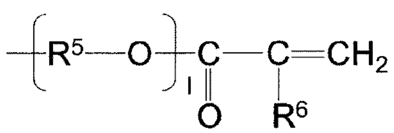

( ii )

where $R^3$ and $R^5$ are each independently a divalent hydrocarbon group having 1 to 10 carbon atoms, $R^4$ and $R^6$ are each independently a hydrogen atom or a methyl group, and k and l are each independently an integer from 0 to 6.

7. The dental restorative curable composition according to claim 6, wherein X is an oxygen atom.

8. The dental restorative curable composition according to claim 6 or 7, wherein k and l are each independently 0 or 1.

**9.** The dental restorative curable composition according to any one of claims 1 to 8, wherein a content of the inorganic filler (C) is 50 to 95 mass% in a total amount of the dental restorative curable composition.

**10.** The dental restorative curable composition according to any one of claims 1 to 9, wherein the polymerization initiator (D) comprises a photopolymerization initiator.

**11.** The dental restorative curable composition according to any one of claims 1 to 10, wherein the polymerization initiator (D) comprises a thermal polymerization initiator.

**12.** A dental composite resin comprising the dental restorative curable composition according to any one of claims 1 to 11.

**13.** A dental mill blank comprising a cured product of the dental restorative curable composition according to any one of claims 1 to 11.

**INTERNATIONAL SEARCH REPORT**

International application No.
**PCT/JP2021/048983**

**A. CLASSIFICATION OF SUBJECT MATTER**

***A61K 6/891***(2020.01)i; ***A61C 13/14***(2006.01)i; ***A61C 13/15***(2006.01)i; ***A61K 6/831***(2020.01)i; ***A61K 6/871***(2020.01)i
FI: A61K6/891; A61C13/14 Z; A61C13/15; A61K6/831; A61K6/871

According to International Patent Classification (IPC) or to both national classification and IPC

**B. FIELDS SEARCHED**

Minimum documentation searched (classification system followed by classification symbols)

A61K6/891; A61C13/14; A61C13/15; A61K6/831; A61K6/871

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN)JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | WO 2020/218446 A1 (KURARAY NORITAKE DENTAL INC) 29 October 2020 (2020-10-29) entire text, all drawings | 1-13 |
| A | JP 5-78435 A (MITSUI PETROCHEM IND LTD) 30 March 1993 (1993-03-30) entire text, all drawings | 1-13 |
| A | JP 62-226907 A (PENTORON CORP) 05 October 1987 (1987-10-05) entire text, all drawings | 1-13 |
| A | WO 2014/123098 A1 (UBE INDUSTRIES, LTD) 14 August 2014 (2014-08-14) entire text, all drawings | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 February 2022** | **08 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/048983**

| Patent document cited in search report | Publication date (day/month/year) | Patent family member(s) | Publication date (day/month/year) |
|---|---|---|---|
| WO 2020/218446 A1 | 29 October 2020 | (Family: none) | |
| JP 5-78435 A | 30 March 1993 | US 5338769 A<br>entire text, all drawings<br>EP 499435 A2<br>CA 2061002 A<br>KR 10-1992-0016549 A<br>CA 2061002 A1 | |
| JP 62-226907 A | 05 October 1987 | US 5444104 A<br>entire text, all drawings<br>US 5276068 A<br>DE 3632868 A<br>CH 674308 A | |
| WO 2014/123098 A1 | 14 August 2014 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S62226907 A
- JP H578435 A
- WO 2014021343 A
- WO 2012042911 A
- JP H93109 A
- JP H10245525 A


### Non-patent literature cited in the description


- *Dental Materials and Devices,* 1988, vol. 7 (5), 756-768
- Fundamentals of Polymer Chemistry (Kobunshi Kagaku No Kiso). 1978